(19) Europäisches Patentamt
European Patent Office
Office européen des brevets

(11) **EP 4 248 868 A1**

(12) **EUROPEAN PATENT APPLICATION**
published in accordance with Art. 153(4) EPC

(43) Date of publication:
**27.09.2023 Bulletin 2023/39**

(21) Application number: **21894584.8**

(22) Date of filing: **15.11.2021**

(51) International Patent Classification (IPC):
***A61B 5/332*** (2021.01)

(52) Cooperative Patent Classification (CPC):
**A61B 5/332**

(86) International application number:
**PCT/JP2021/041854**

(87) International publication number:
**WO 2022/107710 (27.05.2022 Gazette 2022/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.11.2020 JP 2020192638
26.10.2021 JP 2021174265**

(71) Applicant: **Nitto Denko Corporation
Ibaraki-shi, Osaka 567-8680 (JP)**

(72) Inventor: **MINAKATA, Masayuki
Ibaraki-shi, Osaka 567-8680 (JP)**

(74) Representative: **Hoffmann Eitle
Patent- und Rechtsanwälte PartmbB
Arabellastraße 30
81925 München (DE)**

(54) **BIOSENSOR**

(57) A living body sensor according to the present invention is a living body sensor configured to be attached to a living body and obtain a biological signal. The living body sensor includes a housing; a base provided on a living body side of the housing; and an electrode provided at a surface of the base on the living body side. A breaking elongation percentage of the base is 30% to 500%, and a coefficient of static friction of the electrode is 3.0 to 7.0.

FIG.3

EP 4 248 868 A1

**Description**

TECHNICAL FIELD

The present invention relates to a living body sensor.

BACKGROUND ART

A living body sensor that measures biological information such as an electrocardiographic waveform, a pulse wave, a brain wave, or a myoelectric potential is used in a medical institution such as a hospital or a clinic, a nursing facility, or a home. The living body sensor includes a bioelectrode that comes into contact with a living body to obtain biological information of a subject. When the biological information is to be measured, the living body sensor is attached to the skin of the subject and the bioelectrode is brought into contact with the skin of the subject. The biological information is measured by obtaining an electric signal related to the biological information with the bioelectrode.

As such a living body sensor, for example, there is disclosed a biological information measuring garment including a living body contact type electrode in a part of an inner side of the garment, the living body contact type electrode using as a conductive member a stretch conductor sheet containing at least conductive fine particles and a flexible resin binder as ingredients (for example, see Patent Document 1). In the living body contact type electrode attached to the biological information measuring garment, an area of the electrode surface is 1 square centimeter or more, and the coefficient of static friction between the surface of the living body contact type electrode and dry skin is be greater than or equal to 0.4 and smaller than or equal to 2.0.

PRIOR ART DOCUMENT

PATENT DOCUMENT

Patent Document 1: International Publication No. 2019/044649

SUMMARY OF THE INVENTION

PROBLEM TO BE SOLVED BY THE INVENTION

However, in the biological information measurement garment of Patent Document 1, the living body contact type electrode does not have adhesiveness, and the position of the living body contact type electrode is fixed by sandwiching the living body contact type electrode between the skin of the subject and the base of the biological information measurement garment at the time of wearing. For this reason, there is a problem that the position of the living body contact type electrode may be easily displaced due to movement of the garment and movement of the skin of the subject at a time of exercise such as walking and noise may be easily generated in an electrocardiogram at the time of exercise.

An object of the present invention is to provide a living body sensor capable of stably obtaining an electrocardiographic waveform even during exercise.

MEANS FOR SOLVING THE PROBLEM

One aspect of a living body sensor according to the present invention is a living body sensor that is attached to a living body and obtains a biological signal. The living body sensor includes a housing; a base provided on a living body side of the housing; and an electrode provided on a surface of the base on a living body side. A breaking elongation rate of the base is 30% to 500%, and a coefficient of static friction of the electrode is 3.0 to 7.0.

EFFECTS OF THE INVENTION

The aspect of the living body sensor according to the present invention can stably obtain an electrocardiographic waveform even during exercise.

BRIEF DESCRIPTION OF THE DRAWINGS

[FIG. 1] FIG. 1 is a perspective view depicting a configuration of a living body sensor according to an embodiment of the present invention.

[FIG. 2] FIG. 2 is an exploded perspective view of FIG. 1.

[FIG. 3] FIG. 3 is a cross-sectional view taken along line I-I of FIG. 1.

[FIG. 4] FIG. 4 is a plan view depicting a configuration of a sensor unit.

[FIG. 5] FIG. 5 is an exploded perspective view of a portion of the sensor unit of FIG. 3.

[FIG. 6] FIG. 6 is an explanatory view depicting a state in which the living body sensor is attached to the skin of a living body (subject).

[FIG. 7] FIG. 7 is an explanatory diagram depicting an example of an electrocardiographic waveform without noise.

[FIG. 8] FIG. 8 is a diagram illustrating a SN ratio of an electrocardiographic waveform.

MODE FOR CARRYING OUT THE INVENTION

Hereinafter, embodiments of the present invention will be described in detail. In order to facilitate understanding of the description, the same components are denoted by the same reference numerals in the drawings, and redundant description will be omitted. In addition, the scale of each member in the drawings may be different from the actual scale. In the present specification, "to" indicating a numerical range means that the numerical values described before and after the "to" are included as the lower limit value and the upper limit value unless otherwise specified.

<Living Body Sensor>

A living body sensor according to a present embodiment will now be described. A living body refers to a human body (human), an animal such as a cow, a horse, a pig, a chicken, a dog, or a cat, or the like. The living body sensor according to the present embodiment can be suitably used for a living body, particularly for a human body. The living body sensor according to the present embodiment is an attachment type living body sensor that is attached to skin, which is a part of a living body, to measure biological information. With regard to the present embodiment, as an example, a case where the living body is a person will be described.

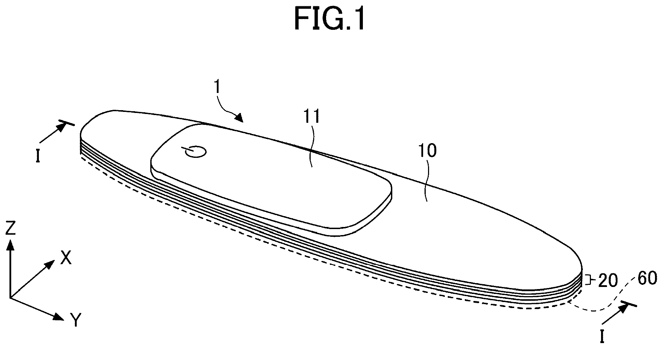

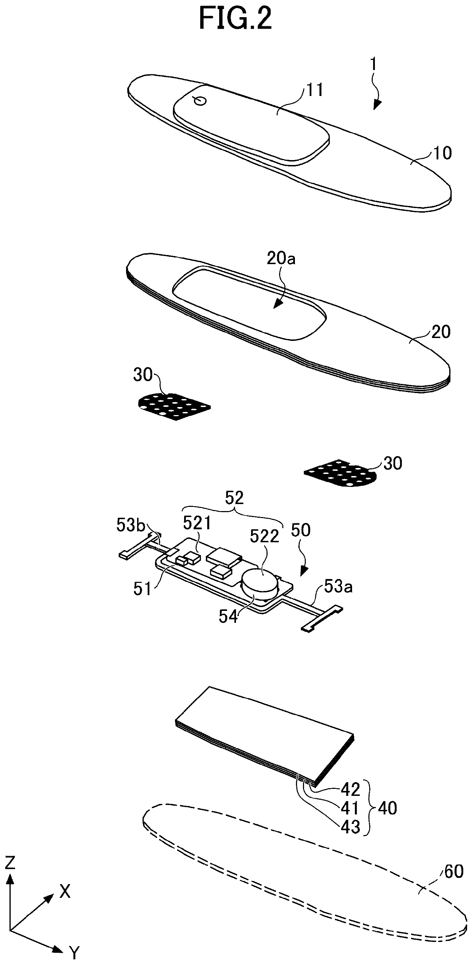

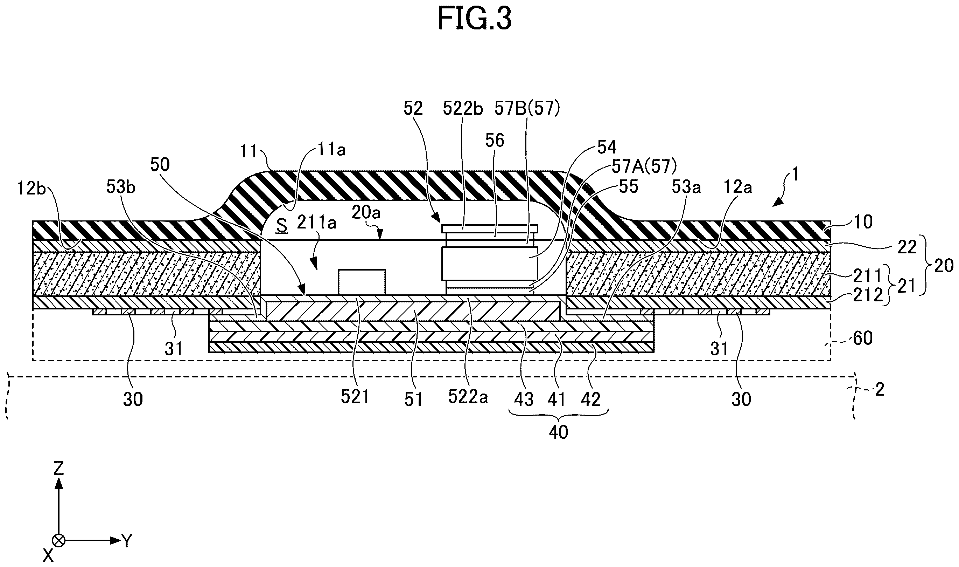

FIG. 1 is a perspective view depicting a configuration of the living body sensor according to the present embodiment, FIG. 2 is an exploded perspective view of FIG. 1, and FIG. 3 is a cross-sectional view taken along line I-I of FIG. 1. As depicted in FIG. 1, the living body sensor 1 is a plate-like (sheet-like) member formed in a substantially elliptical shape in plan view. As illustrated in FIGs. 2 and 3, the living body sensor 1 includes a housing (cover member) 10, a foam sheet 20, electrodes 30, a supporting adhesive sheet 40, and a sensor unit 50, and is formed by stacking the housing 10, the foam sheet 20, the electrodes 30, and the supporting adhesive sheet 40 in this order from the housing 10 side to the supporting adhesive sheet 40 side. The foam sheet 20, the electrodes 30, and the supporting adhesive sheet 40 of the living body sensor 1 are attached to skin 2, which is a living body, and obtains a biological signal from the skin 2 via the electrodes 30. Before the living body sensor 1 is actually used, the living body sensor 1 may have release paper 60 attached onto living-body-attachment-side surfaces of the foam sheet 20, the electrodes 30, and the supporting adhesive sheet 40; when the living body sensor 1 is actually used, the release paper 60 can be removed so that the living body sensor 1 can be attached to the surface of the skin 2.

The housing 10, the foam sheet 20, and the supporting adhesive sheet 40 have substantially the same outer shapes in plan view. The sensor unit 50 is disposed on the supporting adhesive sheet 40, and is housed in a housing space S formed by the housing 10 and the foam sheet 20.

In the present specification, a three dimensional orthogonal coordinate system having three axis directions (X-axis direction, Y-axis direction, and Z-axis direction) is used, and a minor axis direction of the living body sensor is determined as the X-axis direction, a major axis direction thereof is determined as the Y-axis direction, and a height direction (thickness direction) thereof is determined as the Z-axis direction. A direction opposite to a side (attachment side) where the living body sensor is attached to a living body (subject) is referred to as a +Z-axis direction, and a direction of a side (attachment side) where the living body sensor is attached to the living body (subject) is referred to as a -Z-axis direction. In the following description, for convenience of description, a +Z-axis direction side is referred to as an upper side, and a -Z-axis direction side is referred to as a lower side. However, this does not represent the universal vertical relationship.

When making a study for the living body sensor including the foam sheet 20 and the electrode 30, the inventor of the present application focused on a fact that the breaking elongation rate of a foam base 211 included in the foam sheet 20 and the coefficient of static friction $\mu$ of the electrodes 30 relate to suppression of noise generated in an electrocardiogram of the living body sensor 1, and further relate to improvement in durability of the living body sensor 1 and to suppression of a burden to the skin 2 such as skin rash. Then, the inventor of the present application found that when the breaking elongation rate of the foam base 211 is 30% to 500% and the coefficient of static friction $\mu$ of the electrodes 30 is 3.0 to 7.0, occurrence of positional displacement of the living body sensor 1 at the attachment surface of the skin 2 can be suppressed. Thus, the inventor of the present application found that the living body sensor 1 can suppress noise generated in an electrocardiogram even when the subject performs exercise such as walking or moving, and can stably obtain a waveform of an electrocardiogram (electrocardiographic waveform). At the same time, the inventor of the present application found that durability of the living body sensor 1 can be improved and a burden to the skin 2 with the living body sensor 1 can be reduced by setting the breaking elongation rate of the foam base 211 and the coefficient of static friction μ of the electrodes 30 to be within the above-stated ranges.

[Housing]

As depicted in FIGs. 1 to 3, the housing 10 is located on the outermost side (+ Z-axis direction) of the living body sensor 1 and is bonded to an upper surface of the foam sheet 20. The housing 10 has a protruding portion 11 protruding in a substantially dome shape in a height direction (+ Z-axis direction) in FIG. 1 at the center in a longitudinal direction (Y-axis direction), and a recess 11a formed in an indented shape from a living body side is formed on an inner side (attachment side) of the protruding portion 11. In addition, a lower surface (a surface at the attachment side) of the housing 10 is formed to be flat. From the inner side (attachment side) of the protruding portion 11, a housing space S for housing the sensor unit 50 is formed by the recess 11a formed on the inner surface of the protruding portion 11 and a hole 211a of the foam base 211.

As a material of forming the housing 10, for example, a flexible material such as silicone rubber, fluorine rubber, or urethane rubber can be used. In addition, the housing 10 can be formed by using a base resin such as polyethylene terephthalate (PET) as a support and laminating the material having flexibility on a surface of the support. By forming the housing 10 using the above-described material having flexibility and/or the like, it is possible to protect the sensor unit 50 disposed in the housing space S of the housing 10 and absorb an impact applied to the living body sensor 1 from the upper surface side to soften the impact applied to the sensor unit 50.

The thicknesses of an upper wall and the side walls of the protruding portion 11 of the housing 10 are thicker than the thicknesses of flat portions 12a and 12b provided at both end sides in the longitudinal direction (Y-axis direction) of the housing 10. Accordingly, the flexibility of the protruding portion 11 can be made lower than the flexibility of the flat portions 12a and 12b, and the sensor unit 50 can be protected from an external force applied to the living body sensor 1.

It is preferable that the thicknesses of the upper and wall the side walls of the protruding portion 11 are 1.5 mm to 3 mm, and the thicknesses of the flat portions 12a and 12b are 0.5 mm to 1 mm.

Since the thin flat portions 12a and 12b are more flexible than the protruding portion 11, when the living body sensor 1 is attached to the skin 2, the flat portions 12a and 12b can be easily deformed following deformation of the skin 2 caused by exercise such as stretching, bending, or twisting. As a result, stresses applied to the flat portions 12a and 12b when the skin 2 is deformed can be moderated, and the living body sensor 1 can be prevented from being easily peeled off from the skin 2.

Outer peripheral portions of the flat portions 12a and 12b have shapes whose thicknesses gradually decrease toward the ends. Accordingly, it is possible to further increase the flexibility of the outer peripheral portions of the flat portions 12a and 12b, and it is possible to improve a person's feeling of wearing when the living body sensor 1 is attached to the skin 2 compared to a case where the thicknesses of the outer peripheral portions of the flat portions 12a and 12b did not decrease.

The housing 10 preferably has a hardness (strength) of 40 to 70, more preferably 50 to 60. When the hardness of the housing 10 is within the above-described preferable range, the foam sheet 20 can be deformed in accordance with movement of the skin 2 without being affected by the housing 10 when the skin 2 is stretched by exercise. Note that the hardness means Shore A hardness.

[Foam Sheet]

As depicted in FIG. 3, the foam sheet 20 is bonded to the lower surface of the housing 10. The foam sheet 20 has a through hole 20a at a position facing the protruding portion 11 of the housing 10. Thanks to the through hole 20a, a sensor body 52 of the sensor unit 50 is housed in the housing space S formed by the recess 11a on the inner surface of the housing 10 and the through hole 20a without being blocked by the foam sheet 20.

The foam sheet 20 includes a foam attachment layer 21 and a housing adhesive layer 22 that is provided on a surface at the housing 10 side (+Z-axis direction).

(Foam Attachment Layer)

As depicted in FIG. 3, the foam attachment layer 21 includes a foam base (also referred to as a foam) 211 and a base adhesive layer 212 provided on a surface of the foam base 211 at the living body side (-Z-axis direction).

((Foam Base))

The foam base 211 has a porous structure, and can be formed using a porous body having flexibility, waterproofness, and moisture permeability. As the porous body, for example, a foam material of open cells, closed cells, semi-closed cells, or the like can be used. Thus, water vapor produced from sweat or the like produced by the skin 2 to which the living body sensor 1 is attached can be allowed to pass to the outside of the living body sensor 1 through the foam base 211.

The breaking elongation rate of the foam base 211 is 30% to 500%, preferably 40% to 400%, and more preferably 50% to 300%. When the breaking elongation rate of the foam base 211 is less than 30%, the living body sensor cannot deform following movement of the skin 2, noise generated in an electrocardiographic waveform increases, and the subject is likely to feel uncomfortable. When the breaking elongation rate of the foam base 211 exceeds 500%, the volume of the pores formed inside the foam base 211 is large and the foam base 211 is too soft, so that the shape of the foam base 211 is unstable. In addition, since a liquid such as water or sweat easily enters the pores in the foam base 211 and a gap between the foam base 211 and the base adhesive layer 212 or the housing adhesive layer 22, durability of the foam base 211 is likely to be reduced. When the breaking elongation rate of the foam base 211 is 30% to 500%, the living body sensor 1 easily extends in a state of being in contact with the skin 2, and the state of being in contact with the skin 2 can be maintained. Therefore, it is possible to reduce noise generated in an electrocardiographic waveform and to reduce discomfort given to the subject. In addition, since it is possible to suppress intrusion of liquid into the pores in the foam base 211 and the gap between the foam base 211 and the base adhesive layer 212 or the housing adhesive layer 22, it is possible to maintain durability of the foam base 211.

The breaking elongation rate of the foam base 211 means the ratio of the length of the foam base 211 in the major axis direction or the minor axis direction at a time of breaking to the length of the foam base 211 in the major axis direction or the minor axis direction before being pulled, as depicted in the following formula (1).

```
breaking elongation rate of foam base 211
= (length of foam base 211 in major axis direction or minor
axis direction at a time of breaking)/(length of foam base
211 in major axis direction or minor axis direction before
being pulled)  ... (1)
```

The breaking elongation rate of the foam base 211 can be measured using a tensile tester (AGS-J, manufactured by Shimadzu Corporation). The tensile test conditions at this time can be set appropriately. For example, the tensile test can be performed with a tensile strength of 300 mm/min, where 10 mm is the width of the foam base 211 (the maximum length in the minor axis direction of the foam base 211), and 50 mm is the initial distance between a pair of jigs gripping both end portions of the foam base 211. The breaking elongation rate of the foam base 211 may be the breaking elongation rate in either the major axis direction or the minor axis direction of the foam base 211, but from the viewpoint of ease of measurement, the breaking elongation rate in the major axis direction of the foam base 211 is preferable. In the measurement of the breaking elongation rate of the foam base 211, a rectangular sheet of the foam base 211 having a predetermined size (for example, short side 10 mm by long side 70 mm by 0.5 mm thick) may be used. When the rectangular sheet is pulled, both end portions of the short sides of the rectangular sheet may be gripped and fixed by the tensile test jigs, and one or both of the pair of jigs may be moved, in such a manner that the tensile strength should be 300 mm/min. Both end portions at the short sides of the rectangular sheet are regions within predetermined ranges (for example, 15 mm or less) from the end faces of the short sides, depending on the size or the like of the rectangular sheet.

The breaking elongation rate of the foam base 211 may be an average value of measured values of a plurality of (for example, three) foam bases 211.

In the present embodiment, the outer shape of the foam base 211 is an elliptical shape, and when the foam base 211 is attached to the skin 2, the foam base 211 is most likely to expand or constrict in the major axis direction. Therefore, the breaking elongation rate of the foam base 211 is preferably the breaking elongation rate in the major axis direction of the foam base 211. When the outer shape of the foam base 211 is rectangular, the breaking elongation rate of the foam base 211 is preferably the breaking elongation rate in the long-side direction of the foam base 211. When the outer shape of the foam base 211 is a square, the breaking elongation rate of the foam base 211 may be the breaking elongation rate in one side direction of the foam base 211. When the outer shape of the foam base 211 is circular, the breaking elongation rate of the foam base 211 may be the breaking elongation rate of a diameter of the foam base 211.

As a material of the foam base 211, for example, a thermoplastic resin such as a polyurethane-based resin, a polystyrene-based resin, a polyolefin-based resin, a silicone-based resin, an acrylic resin, a vinyl chloride-based resin, or a polyester-based resin can be used.

The thicknesses of the foam base 211 can be set appropriately, and can be, for example, 0.5 mm to 1.5 mm.

The foam base 211 has the hole 211a at a position facing the protruding portion 11 of the housing 10. The through hole 20a can be formed as a result of the base adhesive layer 212 and the housing adhesive layer 22 being formed on the area of the surface other than the area of the hole 211a of the foam base 211.

Although the foam attachment layer 21 uses the foam base 211, it is not limited thereto, and a base having no porous structure may be used instead. What is needed for the base is to have the breaking elongation rate of 30% to 500%, and have flexibility, waterproofness, and moisture permeability. When the base has the breaking elongation rate of 30% to 500%, and has flexibility, waterproofness, and moisture permeability, the base easily extends in a state of being in contact with the skin 2, can maintain the state of being in contact with the skin 2, can suppress intrusion of liquid into the gap between the foam base 211 and the base adhesive layer 212 or the housing adhesive layer 22, and can maintain durability. Thus, water vapor produced from sweat or the like generated by the skin 2 to which the living body sensor 1 is attached can be allowed to pass to the outside of the living body sensor 1 through the base.

As the material of the base, for example, a thermoplastic resin such as a polyurethane-based resin, a polystyrene-based resin, a polyolefin-based resin, a silicone-based resin, an acrylic-based resin, a vinyl chloride-based resin, or a polyester-based resin can be used as in the case of the foam base 211.

((Base Adhesive Layer))

As depicted in FIG. 3, the base adhesive layer 212 is provided in such a manner as being attached to the lower surface of the foam base 211, and has a function of bonding the foam base 211 and the base 41 and bonding the foam base 211 and the electrodes 30.

The base adhesive layer 212 preferably has moisture permeability. Water vapor or the like generated by the skin 2 to which the living body sensor 1 is attached can be allowed to pass to the foam base 211 through the base adhesive layer 212. Furthermore, since the foam base 211 has the porous structure as described above, water vapor can be allowed to pass to the outside of the living body sensor 1 via the housing adhesive layer 22. As a result, it is possible to suppress accumulation of sweat or water vapor at the interface between the skin 2 on which the living body sensor 1 is attached and an attachment adhesive layer 42. As a result, it is possible to prevent the adhesive force of the base adhesive layer 212 from being weakened by the moisture accumulated at the interface between the skin 2 and the base adhesive layer 212, and thus, it is possible to prevent the living body sensor 1 from being peeled off from the skin 2.

The moisture permeability of the base adhesive layer 212 is preferably 1 $(g/m^2 \cdot day)$ or more, and more preferably 10 $(g/m^2 \cdot day)$ or more. The moisture permeability of the base adhesive layer 212 is 10000 $(g/m^2 \cdot day)$ or less. If the moisture permeability of the base adhesive layer 212 is 10 $(g/m^2 \cdot day)$ or more, when the attachment adhesive layer 42 is attached to the skin 2, sweat or the like coming from the supporting adhesive sheet 40 can penetrate toward the outside, so that a burden to the skin 2 can be suppressed.

A material forming the base adhesive layer 212 is preferably a material having pressure-sensitive adhesiveness, and the same material as that of the attachment adhesive layer 42 can be used, and it is preferable to use an acrylic pressure-sensitive adhesive.

As the base adhesive layer 212, a double-sided adhesive tape formed of the above-described material can be used. It is possible to improve the waterproof property of the living body sensor 1 and to improve the adhesion strength with the housing 10 when the living body sensor 1 is formed from stacking the housing 10 on the base adhesive layer 212.

On the surface of the base adhesive layer 212, a wavy pattern (web pattern) may be formed in which adhesive provided portions in each of which an adhesive is present and adhesive not provided portions in each of which no adhesive is present are alternately formed. As the base adhesive layer 212, for example, a double-sided adhesive tape having a web pattern formed on its surface can be used. When the base adhesive layer 212 has the web pattern on the surface thereof, the adhesive can be made to present on the protruding portions of the surface and the periphery thereof, and the adhesive can be prevented from being present on the indented portions of the surface and the periphery thereof. Therefore, since both the portions in each of which the adhesive is present and the portions in each of which the adhesive is not present are present on the surface of the base adhesive layer 212, the adhesive can be dotted on the surface of the base adhesive layer 212. The moisture permeability of the base adhesive layer 212 tends to increase as the thickness of the adhesive decreases. Therefore, since the web pattern is formed on the surface of the base adhesive layer 212 and the surface of the base adhesive layer 212 has the portions where the adhesive is partially thin, it is possible to improve the moisture permeability while maintaining the adhesive force as compared with a case where the web pattern is not formed.

Each of the widths of the adhesive provided portions and the adhesive not provided portions can be designed appropriately. For example, each of the widths of the adhesive provided portions is preferably 500 $\mu$m to 1000 pm, and each of the widths of the adhesive not provided portions is preferably 1500 um to 5000 $\mu$m. When the widths of the adhesive provided portions and the adhesion not provided portions each fall within the above-described preferable ranges, the base adhesive layer 212 can exhibit excellent moisture permeability while maintaining the adhesive force.

The thickness of the base adhesive layer 212 can be set appropriately, and is preferably 10 $\mu$m to 300 $\mu$m, more preferably 50 um to 200 $\mu$m, and further preferably 70 $\mu$m to 110 $\mu$m. When the thickness of the base adhesive layer 212 is 10 $\mu$m to 300 um, the thickness of the living body sensor 1 can be reduced.

(Housing Adhesive Layer)

As depicted in FIG. 3, the housing adhesive layer 22 is provided in a state of being attached to the upper surface of the foam base 211. The housing adhesive layer 22 is attached to the upper surface of the foam base 211 at a position corresponding to the flat portions at the attachment side (-Y-axis direction) of the housing 10, and has a function of bonding the foam base 211 and the housing 10.

As a material of forming the housing adhesive layer 22, a silicon-based adhesive silicone tape or the like can be used.

The thickness of the housing adhesive layer 22 can be set appropriately, and can be, for example, 10 um to 300 $\mu$m.

(Electrodes)

As illustrated in FIG. 3, the electrodes 30 are attached to the lower surface of the base adhesive layer 212 at the attachment side (-Z-axis direction) in a state in which parts of the electrodes 30 at the sensor body 52 sides are connected to wires 53a and 53b and are sandwiched between the base adhesive layer 212 and a sensor adhesive layer 43. Portions of the electrodes 30 that are not sandwiched between the base adhesive layer 212 and the sensor adhesive layer 43 come into contact with the living body. When the living body sensor 1 is attached to the skin 2, the electrodes 30 come into contact with the skin 2, thereby detecting a biological signal. The biological signal is, for example, an electrical signal representing an electrocardiographic waveform, a brain wave, a pulse, or the like. Note that the electrodes 30 may be embedded in the base 41 in a state of being exposed so as to be able to contact the skin 2.

The electrodes 30 contain a conductive polymer and a binder resin, and the conductive polymer is contained in a dispersed state in the binder resin.

The electrodes 30 can be formed using electrode sheets in each of which a cured product of a conductive composition containing the conductive polymer and the binder resin, and a metal, an alloy, or the like is formed into a sheet shape.

As the conductive polymer to use, for example, a polythiophene-based conductive polymer, a polyaniline-based conductive polymer, a polypyrrole-based conductive polymer, a polyacetylene-based conductive polymer, a polyphenylene-based conductive polymer, a derivative of each thereof, a complex of two or more thereof, or the like can be used. These may be used alone or in combination of two or more thereof.

Examples of the polythiophene-based conductive polymer include polythiophene, poly(3-methylthiophene), poly(3-ethylthiophene), poly(3-propylthiophene), poly(3-butylthiophene), poly(3-hexylthiophene), poly(3-heptylthiophene), poly(3-octylthiophene), poly(3-decylthiophene), poly(3-dodecylthiophene), poly(3-octadecylthiophene), poly(3-bromothiophene), poly(3-chlorothiophene), poly(3-iodothiophene), poly(3-cyanothiophene), poly(3-phenylthiophene), poly(3,4-dimethylthiophene), poly(3,4-dibutylthiophene), poly(3-hydroxythiophene), poly(3-methoxythiophene), poly(3-ethoxythiophene), poly(3-butoxythiophene), poly(3-hexyloxythiophene), poly(3-heptyloxythiophene), poly(3-octyloxythiophene), poly(3-decyloxythiophene), poly(3-dodecyloxythiophene), poly(3-octadecyloxythiophene), poly(3,4-dihydroxythiophene), poly(3,4-dimethoxythiophene), poly(3,4-diethoxythiophene), poly(3,4-dipropoxythiophene), poly(3,4-dibutoxythiophene), poly(3,4-dihexyloxythiophene), poly(3,4-diheptyloxythiophene), poly(3,4-dioctyloxythiophene), poly(3,4-didecyloxythiophene), poly(3,4-didodecyloxythiophene), poly(3,4-ethylenedioxythiophene) (also referred to as PEDOT), poly(3, 4-propylenedioxythiophene), poly(3,4-butenedioxythiophene), poly(3-methyl-4-methoxythiophene), poly(3-methyl-4-ethoxythiophene), poly(3-carboxythiophene), poly(3-methyl-4-carboxythiophene), poly(3-methyl-4-carboxyethylthiophene), and poly(3-methyl-4-carboxybutylthiophene).

Examples of the polyaniline-based conductive polymer include polymers having sulfonic acid groups of polyaniline, polystyrenesulfonic acid (also referred to as PSS), polyvinylsulfonic acid, polyallylsulfonic acid, polyacrylsulfonic acid, polymethacrylsulfonic acid, poly(2-acrylamide-2-methylpropanesulfonic acid), polyisoprenesulfonic acid, polysulfoethylmethacrylate, poly(4-sulfobutylmethacrylate), polymethacryloxybenzenesulfonic acid, and the like; and polymers having carboxylic acid groups of polyvinylcarboxylic acid, polystyrenecarboxylic acid, polyallylcarboxylic acid, polyacrylcarboxylic acid, polymethacrylcarboxylic acid, poly(2-acrylamide-2-methylpropanecarboxylic acid), polyisoprenecarboxylic acid, polyacrylic acid, and the like. Each thereof may be used as a homopolymer obtained by polymerizing the one thereof alone, or may be used as a copolymer of two or more thereof. Among these polyanilines, a polymer having a sulfonic acid group is preferable, and polystyrene sulfonic acid is more preferable, because conductivity can be further increased.

Examples of the polypyrrole-based conductive polymer include polypyrrole, poly(N-methylpyrrole), poly(3-methylpyrrole), poly(3-ethylpyrrole), poly(3-n-propylpyrrole), poly(3-butylpyrrole), poly(3-octylpyrrole), poly(3-decylpyrrole), poly(3-dodecylpyrrole), poly(3,4-dimethylpyrrole), poly(3,4-dibutylpyrrole), poly(3-carboxypyrrole), poly(3-methyl-4-carboxypyrrole), poly(3-methyl-4-carboxyethylpyrrole), poly(3-methyl-4-carboxybutylpyrrole), poly(3-hydroxypyrrole), poly(3-methoxypyrrole), poly(3-ethoxypyrrole), poly(3-butoxypyrrole), poly(3-hexyloxypyrrole), and poly(3-methyl-4-hexyloxypyrrole).

Examples of the polyacetylene-based conductive polymer include polyacetylenes having polar groups such as a polyphenylacetylene monoester having an ester at a para-position of the phenylacetylene and a polyphenylacetylene monoamide having an amide at a para-position of the phenylacetylene.

Examples of the polyphenylene conductive polymer include polyphenylene vinylene and the like.

Examples of the complex of two or more thereof include a complex in which polythiophene is doped with polyaniline as a dopant. PEDOT/PSS or the like in which PEDOT is doped with PSS can be used as the complex of polythiophene and polyaniline.

Among the above conductive polymers, the complex in which polythiophene is doped with polyaniline as a dopant is preferable. Among the complexes of polythiophenes and polyanilines, PEDOT/PSS in which PEDOT is doped with PSS is more preferable in terms of its lower contact impedance with a living body and its high conductivity.

In addition, it is preferable that the electrodes 30 have a plurality of through holes 31 formed through the contact surface with the skin 2. As a result, in a state where the electrodes 30 are attached to the base adhesive layer 212, the base adhesive layer 212 can be exposed through the through holes 31 to the attachment side, and thus adhesion between the electrodes 30 and the skin 2 can be improved.

The coefficient of static friction $\mu$ of the electrodes 30 is 3.0 to 7.0, preferably 3.5 to 6.5, and more preferably 4.0 to 6.0. When the coefficient of static friction $\mu$ of the electrodes 30 is less than 3.0, the tack of the electrodes 30 is small so the electrodes 30 are easily displaced from the skin 2. Therefore, noise generated in an electrocardiographic waveform measured by the living body sensor 1 is likely to increase. If the coefficient of static friction $\mu$ of the electrodes 30 exceeds 7.0, the adhesion of the electrodes 30 to the skin 2 is too high. Therefore, when the living body sensor 1 is adhered to the skin 2 for a long time (for example, 24 hours), a burden to the skin 2 such as skin chapping is likely to increase. When the coefficient of static friction $\mu$ of the electrodes 30 is within the above-described preferable range, the electrodes 30 can have appropriate tack, and thus the adhesion with respect to the skin 2 can be maintained. Therefore, it is possible to prevent the electrodes 30 from easily being displaced from the skin 2. In addition, since the electrodes 30 can be made to adhere to the skin 2 with the appropriate adhesive force, it is possible to reduce a burden to the skin 2.

The coefficient of static friction $\mu$ is a ratio of a maximum frictional force applied to a contact surface to a reaction force perpendicular to the contact surface when two objects at rest are in contact with each other.

In the present embodiment, the coefficient of static friction $\mu$ of the electrodes 30 can be measured using a coefficient of static friction measuring device (TRIBOGEAR, type: 10, manufactured by SHINTO Scientific Co., Ltd.). The coefficient of static friction $\mu$ of the electrode 30 with respect to a pseudo skin can be measured as follows: As the pseudo skin, a BIOSKIN plate (manufactured by Beaulax Co., Ltd., product number P001-001, longitudinal size 195 mm by lateral size 120 mm by thick size 5 mm) or the like in which hydrophilicity and hydrophobicity and surface wrinkles similar to those of dry human skin are reproduced by processing a surface of an urethane elastomer film may be used. Then, the BIOSKIN plate is fixed to an elevating plate in a horizontal state, the electrode 30 is attached to a planar indenter having a predetermined size (for example, vertical size 75 mm by horizontal size 35 mm), and the plate is kept standing for 30 seconds under the condition of a load of 1.47 N. Then, the plate is inclined at an elevating speed of 10 degrees/6 seconds on average, and the friction angle $\theta d$ when the planar indenter starts to slide is read. By substituting the read friction angle $\theta d$ in the following formula (I), the coefficient of static friction $\mu$ is obtained.

$$\text{coefficient of static friction } \mu$$
$$= \tan(\theta d \times \pi / 180) \ \dots \ (I)$$

[Supporting Adhesive Sheet]

As depicted in FIG. 3, the supporting adhesive sheet 40 includes the base 41, the attachment adhesive layer 42, and the sensor adhesive layer 43.

(Second Base)

As depicted in FIG. 3, the base 41 is such that the contour shapes on both sides in the width direction (X-axis direction) of the attachment adhesive layer 42 is substantially the same as the contour shapes on both sides in the width direction (X-axis direction) of the housing 10 and the foam sheet 20. The length (Y-axis direction) of the base 41 is formed to be shorter than the length (Y-axis direction) of the housing 10 and the foam sheet 20. Both ends of the supporting adhesive sheet 40 in the longitudinal direction are at positions where the wires 53a and 53b of the sensor unit 50 are sandwiched between the supporting adhesive sheet 40 and the foam sheet 20, and are at positions of overlapping partially with the electrodes 30. The sensor adhesive layer 43 is provided on the upper surface of the base 41, and the base adhesive layer 212 is provided on the attachment surface of the foam sheet 20. The sensor adhesive layer 43 of the supporting adhesive sheet 40 and the base adhesive layer 212 of the foam sheet 20 that further extends from both ends in the longitudinal direction of the supporting adhesive sheet 40 form an attachment surface with respect to the skin 2. Accordingly, waterproofness and moisture permeability are different and adhesiveness is different depending on the position of the attachment surface, but, in terms of the entire living body sensor 1, the adhesiveness in the attachment surface corresponding to the foam sheet 20 greatly affects the adhesive performance with respect to the skin 2.

The base 41 can be formed using a flexible resin having appropriate stretchability, flexibility, and toughness. Examples of a material of forming the base 41 include thermoplastic resins such as polyester resins such as polyethylene terephthalate (PET), polybutylene terephthalate, polytrimethylene terephthalate, polyethylene naphthalate, and polybutylene naphthalate; acrylic resins such as polyacrylic acid, polymethacrylic acid, polymethyl acrylate, polymethyl methacrylate (PMMA), polyethyl methacrylate, and polybutyl acrylate; polyolefin resins such as polyethylene and polypropylene; polystyrene resins such as polystyrene, imide-modified polystyrene, acrylonitrile-butadiene-styrene (ABS) resin, imide-modified ABS resin, styrene-acrylonitrile copolymer (SAN) resin, and acrylonitrile-ethylene-propylene-diene-styrene (AES) resin; polyimide resins; polyurethane resins; silicone resins; and polyvinyl chloride resins such as polyvinyl chloride and vinyl chloride-vinyl acetate copolymer resins. Among them, the polyolefin resins and PET are preferable. These thermoplastic resins have waterproof properties (low moisture permeability). Therefore, by forming the base 41 using any of these thermoplastic resins, it is possible to prevent intrusion of sweat or water vapor generated from the skin 2 into the flexible substrate 51 side of the sensor unit 50 through the base 41 in a state where the living body sensor 1 is attached to the skin 2 of a living body.

Since the sensor unit 50 is installed on the upper surface of the base 41, the base 41 is preferably formed in a flat plate shape.

The thickness of the base 41 can be freely and appropriately selected. For example, the thickness is preferably 1 $\mu$m to 300 pm, more preferably 5 $\mu$m to 100 pm, and still more preferably 10 $\mu$m to 50 $\mu$m.

(Attachment Adhesive Layer)

As depicted in FIG. 3, the attachment adhesive layer 42 is provided on the lower surface at the attachment side (-Z-axis direction) of the base 41, and is a layer that comes into contact with a living body.

The attachment adhesive layer 42 preferably has pressure-sensitive adhesiveness. When the attachment adhesive layer 42 has pressure-sensitive adhesiveness, the living body sensor 1 can be easily made to be attached to the skin 2 of a living body as a result of the living body sensor 1 being pressed onto the skin 2.

The material of the attachment adhesive layer 42 is not particularly limited as long as it is a material having pressure-sensitive adhesiveness, and examples thereof include a material having biocompatibility. Examples of the material of forming the attachment adhesive layer 42 include an acrylic pressure-sensitive adhesive and a silicone pressure-sensitive adhesive. Preferably, the acrylic pressure-sensitive adhesive is used.

The acrylic pressure-sensitive adhesive preferably contains an acrylic polymer as a principal ingredient. The acrylic polymer can function as a pressure-sensitive adhesive ingredient. As the acrylic polymer, a polymer obtained by polymerizing a monomer ingredient containing a (meth)acrylic acid ester such as isononyl acrylate or methoxyethyl acrylate as a principle ingredient and a monomer copolymerizable with the (meth)acrylic acid ester such as acrylic acid as an optional ingredient can be used.

The acrylic pressure-sensitive adhesive preferably further contains a carboxylic acid ester. The carboxylic acid ester functions as a pressure-sensitive adhesive force adjusting agent that reduces the pressure-sensitive adhesive force of the acrylic polymer to adjust the pressure-sensitive adhesive force of the attachment adhesive layer 42. As the carboxylic acid ester, a carboxylic acid ester compatible with acrylic polymer can be used. As the carboxylic acid ester, glyceryl tri-fatty acid or the like can be used.

The acrylic pressure-sensitive adhesive may contain a crosslinking agent if necessary. The crosslinking agent is a crosslinking ingredient that crosslinks the acrylic polymer. Examples of the crosslinking agent include polyisocyanate compounds (polyfunctional isocyanate compounds), epoxy compounds, melamine compounds, peroxide compounds, urea compounds, metal alkoxide compounds, metal chelate compounds, metal salt compounds, carbodiimide compounds, oxazoline compounds, aziridine compounds, and amine compounds. Among them, the polyisocyanate compounds are preferable. These crosslinking agents may be used alone or in combination.

The attachment adhesive layer 42 preferably has excellent biocompatibility. For example, when the attachment adhesive layer 42 is subjected to a keratin peeling test, the keratin peeling area ratio is preferably 0% to 50%, and more preferably 1% to 15°. If the keratin peeling area ratio is in the range of 0% to 50%, a burden to skin 2 can be suppressed when the attachment adhesive layer 42 is attached to the skin 2.

The attachment adhesive layer 42 preferably has moisture permeability. Water vapor or the like generated from skin 2 to which the living body sensor 1 is attached is allowed to pass to the foam sheet 20 side through the attachment adhesive layer 42. Furthermore, since the foam sheet 20 has a porous structure as will be described later, water vapor is allowed to pass to the outside of the living body sensor 1 through the attachment adhesive layer 42. As a result, it is possible to suppress accumulation of sweat or water vapor at the interface between the skin 2 on which the living body sensor 1 is attached and the attachment adhesive layer 42. As a result, the adhesive force of the attachment adhesive layer 42 is prevented from being weakened by moisture accumulated at the interface between the skin 2 and the attachment adhesive layer 42, and therefore, the living body sensor 1 can be prevented from being peeled off from the skin.

The moisture permeability of the attachment adhesive layer 42 is preferably 300 (g/m²·day) or more, more preferably 600 (g/m²·day) or more, and further preferably 1000 (g/m²·day) or more. The moisture permeability of the attachment adhesive layer 42 is 10000 (g/m²·day) or less. When the moisture permeability of the attachment adhesive layer 42 is 300 (g/m²·day) or more, when the attachment adhesive layer 42 is attached to skin 2, sweat or the like generated from the skin 2 can be appropriately allowed to pass from the base 41 to the outside, and thus a burden to the skin 2 can be reduced.

The thickness of the attachment adhesive layer 42 can be selected appropriately and is preferably 10 $\mu$m to 300 um. When the thickness of the attachment adhesive layer 42 is 10 $\mu$m to 300 pm, the thickness of the living body sensor 1 can be reduced.

(Sensor Adhesive Layer)

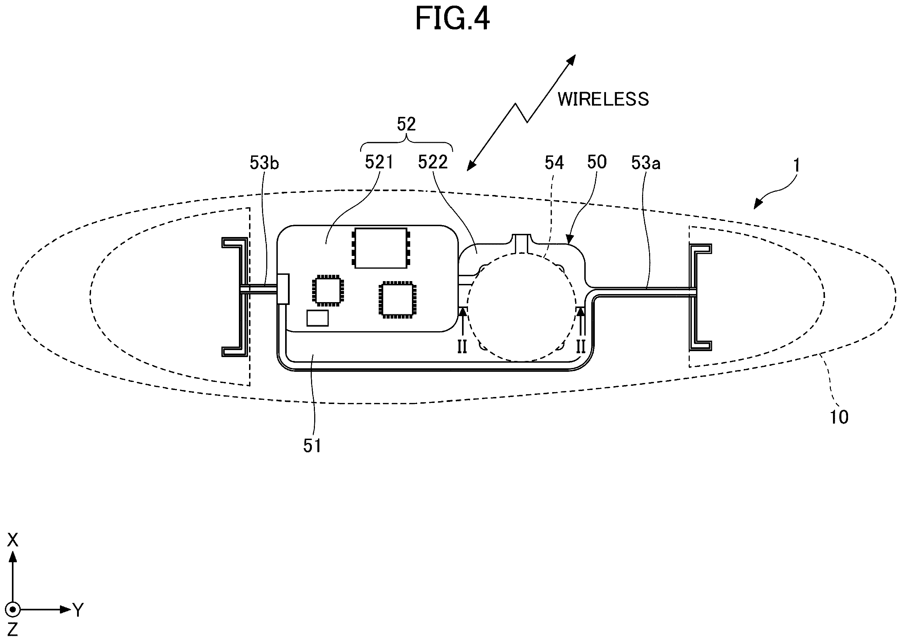

As illustrated in FIG. 4, the sensor adhesive layer 43 is provided on the upper surface of the base 41 at the housing 10 side (+Z-axis direction), and is a layer to which the sensor unit 50 is bonded. The sensor adhesive layer 43 can be made of the same material as that of the attachment adhesive layer 42, and thus will not be described in detail here. Note that the sensor adhesive layer 43 is not necessarily provided, and instead of the sensor adhesive layer 43, an adhesive may be provided on a part or the entirety of the base 41.

(Sensor Unit)

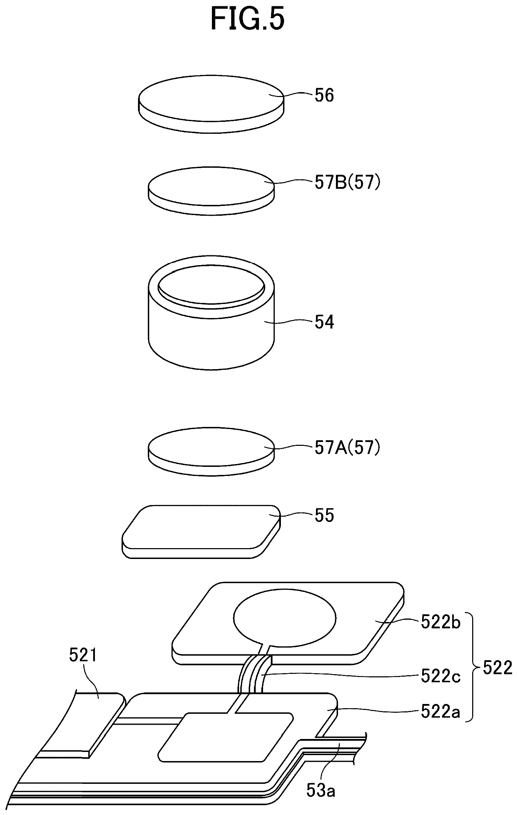

FIG. 4 is a plan view depicting a configuration of the sensor unit 50, and FIG. 5 is an exploded perspective view of a part of the sensor unit 50. The broken line in FIG. 4 indicates a contour of the housing 10. As depicted in FIGs. 4 and 5, the sensor unit 50 includes a flexible substrate 51 on which various components for obtaining biological information are mounted, a sensor body 52, wires 53a and 53b connected to the sensor body 52 in the longitudinal direction thereof, a battery 54, a positive electrode pattern 55, a negative electrode pattern 56, and conductive adhesive tapes 57. Between a pad portion 522a and a pad portion 522b of the sensor unit 50, the positive electrode pattern 55, the conductive adhesive tape 57, the battery 54, the conductive adhesive tape 57, and the negative electrode pattern 56 are stacked in this order from the pad portion 522a side to the pad portion 522b side. In the present embodiment, a positive electrode terminal of the battery 54 is set in the -Z-axis direction and a negative electrode terminal is set in the +Z-axis direction. However, reversely, the positive electrode terminal may be set in the +Z-axis direction and the negative electrode terminal may be set in the -Z-axis direction.

The flexible substrate 51 is a resin substrate, and the sensor body 52 and the wires 53a and 53b are integrally formed on the flexible substrate 51.

As depicted in FIG. 3, one end of each of the wires 53a and 53b is coupled to a corresponding one of the electrodes 30. As depicted in FIG. 4, the other end of the wire 53a is connected to a switch or the like mounted in a component mounting unit 521 along an outer periphery of the sensor body 52. The other end of the wire 53b is also connected to a switch or the like mounted in the component mounting unit 521 similarly to the wire 53a. The wires 53a and 53b may be formed in either of wire layers of the front surface side and the back surface side of the flexible substrate 51.

As depicted in FIG. 4, the sensor body 52 includes the component mounting unit 521, which is a control unit, and a battery mounting unit 522.

The component mounting unit 521 includes various components mounted on the flexible substrate 51, such as a CPU and an integrated circuit that process a biological signal obtained from a living body and generate biological signal data, a switch that activates the living body sensor 1, a flash memory that stores a biological signal, and a light emitting element. Note that circuit examples of the various components are omitted from FIG. 4. The component mounting unit 521 is operated by electric power supplied from the battery 54 loaded in the battery mounting unit 522.

The component mounting unit 521 performs wired or wireless signal transmission to external devices such as an operation check device that checks an initial operation, a reading device that reads biological information from the living body sensor 1, and the like.

The battery mounting unit 522 supplies power to the integrated circuit and the like mounted in the component mounting unit 521. As depicted in FIG. 2, the battery 54 is loaded in the battery mounting unit 522.

As depicted in FIG. 5, the battery mounting unit 522 is disposed between the wire 53a and the component mounting unit 521, and includes the pad portions 522a and 522b and a constricted portion 522c.

As depicted in FIG. 5, the pad portion 522a is provided between the wire 53a and the component mounting unit 521, is positioned at the positive electrode terminal side of the battery 54, and has the positive electrode pattern 55 to which the positive electrode terminal is connected.

As depicted in FIG. 5, the pad portion 522b is provided at a predetermined distance from the pad portion 522a in a direction (upward direction in FIG. 3) orthogonal to the longitudinal direction of the pad portion 522a. The pad portion 522b has the negative electrode pattern 56 which is located at the negative electrode (second electrode) side of the battery 54 and to which the negative electrode is connected.

As depicted in FIG. 5, the constricted portion 522c is disposed between the pad portions 522a and 522b and connects the pad portions 522a and 522b to each other.

As depicted in FIG. 5, the battery 54 is disposed between the positive electrode pattern 55 and the negative electrode pattern 56. The battery 54 has the positive electrode terminal and the negative electrode terminal, and a known battery can be used as the battery 54. As the battery 54, for example, a coin-type battery such as that of CR2025 can be used.

As depicted in FIG. 5, the positive electrode pattern 55 is located on the positive electrode terminal side of the battery 54 and is connected to the positive electrode terminal. The positive electrode pattern 55 has a rectangular shape with its corners chamfered.

As depicted in FIG. 5, the negative electrode pattern 56 is located on the negative electrode terminal side of the battery 54 and connected to the negative electrode terminal. The negative electrode pattern 56 has a shape substantially corresponding to the size of a circular shape of the negative electrode terminal of the battery 54. The diameter of the negative electrode pattern 56 is, for example, equal to a diameter of the battery 54 and substantially equal to the length of a diagonal line of the positive electrode pattern 55.

The conductive adhesive tapes 57 are adhesives having conductivity, and are disposed between the battery 54 and the positive electrode pattern 55 and between the battery 54 and the negative electrode pattern 56, respectively. The conductive adhesive tapes may be generally referred to as conductive adhesive sheets, conductive adhesive films, or the like.

When the battery 54 is to be loaded in the living body sensor 1, the conductive adhesive tape 57A and the conductive adhesive tape 57B are attached to the entire positive electrode pattern 55 and the entire negative electrode pattern 56, respectively. Then, the positive electrode terminal and the negative electrode terminal of the battery 54 are respectively attached to the positive electrode pattern 55 and the negative electrode pattern 56 via the conductive adhesive tape 57A and the conductive adhesive tape 57B, so that the battery 54 is loaded in the battery mounting unit 522. The sensor body 52 depicted in FIG. 4 is a state in which the constricted portion 522c is bent and the battery 54 is loaded in the battery mounting unit 522 in a state of being sandwiched between the positive electrode pattern 55 and the negative electrode pattern 56.

As depicted in FIG. 3, the living body sensor 1 is such that, in order to protect the base 41 and the electrodes 30, it is preferable that release paper 60 is attached to the attachment surface side (-Z-axis direction) until the living body sensor 1 is attached to skin 2. Thus, as a result of the release paper 60 being peeled off from the base 41 and the electrodes 30 at a time of use, the adhesive strength of the base 41 can be maintained until then.

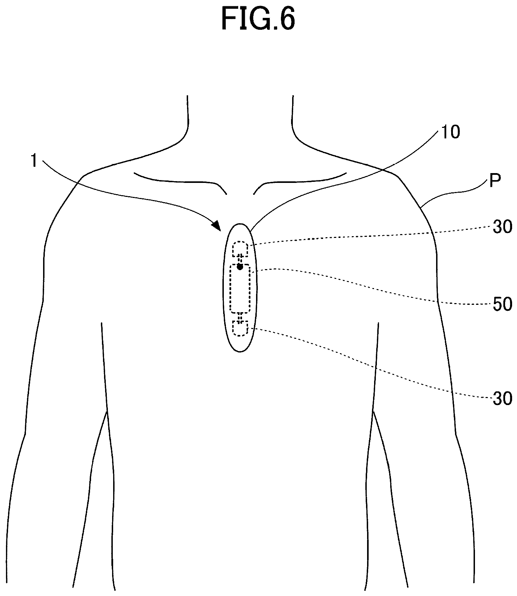

FIG. 6 is an explanatory view depicting a state in which the living body sensor 1 of FIG. 1 is attached to a chest of a living body P. For example, the living body sensor 1 is attached to the skin of the subject P in such a manner that the longitudinal direction (Y-axis direction) is aligned with the sternum of the subject P, one electrode 30 is at the upper side, and the other electrode 30 is at the lower side. The living body sensor 1 obtains a biological signal such as an electrocardiographic signal from the subject P through the electrodes 30 in a state where the electrodes 30 are pressed onto the skin of the subject P as a result of being attached to the skin of the subject P with the attachment adhesive layer 42 of FIG. 2. The living body sensor 1 stores the obtained biological signal data in a nonvolatile memory such as a flash memory mounted in the component mounting unit 521.

A method of manufacturing the living body sensor 1 is not particularly limited, and the living body sensor 1 can be manufactured using any appropriate method. An example of a method of manufacturing the living body sensor 1 will now be described.

The housing 10, the foam sheet 20, the electrodes 30, the supporting adhesive sheet 40, and the sensor unit 50 depicted in FIG. 1 are prepared. Specific methods of manufacturing the housing 10, the foam sheet 20, and the supporting adhesive sheet 40 are not particularly limited as long as they can be manufactured by these methods, and these products can be manufactured in any manufacturing method.

Similarly to the housing 10, the foam sheet 20, and the supporting adhesive sheet 40, a specific method of manufacturing the electrodes 30 is not particularly limited as long as the electrodes 30 can be manufactured by the method, and the electrodes 30 can be manufactured in any manufacturing method. For example, the electrodes 30 can be made of a conductive composition.

The conductive composition will now be described. The conductive composition contains a conductive polymer and a binder resin, and the conductive polymer is contained in a state of being dispersed in the binder resin.

Since the conductive polymer is the same as that described above, the details thereof are omitted here.

The content of the conductive polymer is preferably 0.20 parts by mass to 20 parts by mass, more preferably 2.5 parts by mass to 15 parts by mass, and still more preferably 3.0 parts by mass to 12 parts by mass with respect to 100 parts by mass of the conductive composition. When the content is within the preferable range described above with respect to the conductive composition, the conductive composition can have excellent conductivity, toughness, and flexibility.

The conductive polymer may be of a solid material formed into a pellet shape. In this case, part of the solid material of the conductive polymer may be dissolved in a solvent to such an extent that the content of each ingredient with respect to the conductive composition does not largely vary.

The conductive polymer may be used as an aqueous solution obtained by dissolving the conductive polymer in a solvent. In this case, either an organic solvent or an aqueous solvent can be used as the solvent. Examples of the organic solvent include ketones such as acetone and methyl ethyl ketone (MEK); esters such as ethyl acetate; ethers such as propylene glycol monomethyl ether; and amides such as N,N-dimethylformamide. Examples of the aqueous solvent include water; and alcohols such as methanol, ethanol, propanol, and isopropanol. Among them, the aqueous solvents are preferable.

As the binder resin, a water-soluble polymer or a water-insoluble polymer can be used. As the binder resin, a water-soluble polymer is preferably used from the viewpoint of compatibility with other ingredients contained in the conductive composition. The water-soluble polymer may be a polymer that is not completely dissolved in water and has hydrophilicity (hydrophilic polymer).

As the water-soluble polymer, a hydroxyl group-containing polymer or the like can be used. Examples of the hydroxyl group-containing polymer include saccharides such as agarose, polyvinyl alcohol (PVA), modified polyvinyl alcohol, polypyrrole, and a copolymer of acrylic acid and sodium acrylate. These may be used alone or in combination of two or more thereof. Among them, polyvinyl alcohol or modified polyvinyl alcohol is preferable, and modified polyvinyl alcohol is more preferable.

Examples of the modified polyvinyl alcohol include acetoacetyl group-containing polyvinyl alcohol and diacetoneacrylamide-modified polyvinyl alcohol. As the diacetoneacrylamide-modified polyvinyl alcohol, for example, a diacetoneacrylamide-modified polyvinyl alcohol-based resin (DA-modified PVA-based resin) described in Japanese Patent Application Publication No. 2016-166436 may be used.

Examples of the polypyrrole include polyaniline and polyacetylene.

The content of the binder resin is preferably 5 parts by mass to 140 parts by mass, more preferably 10 parts by mass to 100 parts by mass, and still more preferably 20 parts by mass to 70 parts by mass with respect to 100 parts by mass of the conductive composition. When the content is within the above preferable range with respect to the conductive composition, a cured product obtained using the conductive composition can have excellent conductivity, toughness, and flexibility.

The binder resin may be used as an aqueous solution in which the binder resin is dissolved in a solvent. As the solvent, the same solvent as that in the case of the conductive polymer described above may be used.

The conductive composition preferably further contains at least one of a crosslinking agent or a plasticizer. Each of the crosslinking agent and the plasticizer has a function of imparting toughness and flexibility to a cured product obtained by using the conductive composition.

The toughness is a property including both excellent strength and an excellent degree of elongation. The toughness should not be a property in which one of strength and a degree of elongation is remarkably excellent but the other is remarkably low, and may be a property in which a balance between strength and a degree of elongation is excellent.

The flexibility is a property of suppressing occurrence of damage such as breakage at a bent portion after a cured product containing the conductive composition is bent.

The crosslinking agent has a function of crosslinking the binder resin. When the crosslinking agent is contained in the binder resin, the toughness of a cured product obtained by using the conductive composition can be improved. The crosslinking agent is preferably reactive with a hydroxyl group. If the crosslinking agent has reactivity with a hydroxyl group, when the binder resin is a hydroxyl group-containing polymer, the crosslinking agent can react with the hydroxyl group of the hydroxyl group-containing polymer.

Examples of the crosslinking agent include zirconium compounds such as zirconium salts; titanium compounds such as titanium salts; boron compounds such as a boric acid; isocyanate compounds such as a blocked isocyanate; aldehyde compounds such as sodium glyoxylate, formaldehyde, acetaldehyde, glyoxal, and glutaraldehyde; alkoxyl group-containing compounds; and methylol group-containing compounds. These may be used alone or in combination of two or more thereof. Among them, when the binder resin is polyvinyl alcohol, sodium glyoxylate is preferable from the viewpoint of easily forming a crosslinked structure by reacting with the polyvinyl alcohol and easily maintaining the performance of the cured product obtained by using the conductive composition.

Since the crosslinking agent is an optional ingredient, it is not necessarily contained in the conductive composition, and the content of the crosslinking agent may be 0 parts by mass. When the conductive composition contains the crosslinking agent, the content of the crosslinking agent is preferably 0.01 parts by mass to 1.5 parts by mass, more preferably 0.2 parts by mass to 1.2 parts by mass, and still more preferably 0.4 parts by mass to 1.0 parts by mass with respect to 100 parts by mass of the conductive composition. When the content is within the preferable range described above, a cured product obtained using the conductive composition can have excellent toughness and flexibility.

The crosslinking agent may be used as an aqueous solution obtained by dissolving the crosslinking agent in a solvent. As the solvent, the same solvent as that in the case of the conductive polymer described above can be used.

The plasticizer has a function of improving the conductivity of a cured product obtained by using the conductive composition, and also, improving the degree of tensile elongation and flexibility. Examples of the plasticizer include polyol compounds such as glycerin, ethylene glycol, propylene glycol, sorbitol, and polymers thereof; and aprotic compounds such as N-methylpyrrolidone (NMP), dimethyl formaldehyde (DMF), N-N'-dimethylacetamide (DMAc), and dimethyl sulfoxide (DMSO). These may be used alone or in combination of two or more kinds thereof. Among them, glycerin is preferable from the viewpoint of compatibility with another ingredient.

The content of the plasticizer is preferably 0.2 parts by mass to 150 parts by mass, more preferably 1.0 parts by mass to 90 parts by mass, and still more preferably 10 parts by mass to 70 parts by mass with respect to 100 parts by mass of the conductive composition. When the content is within the preferable range described above, a cured product obtained using the conductive composition can have excellent toughness and flexibility.

When the conductive composition contains at least one of the crosslinking agent or the plasticizer, a cured product obtained using the conductive composition can be improved in toughness and flexibility.

When the conductive composition contains the crosslinking agent but does not contain the plasticizer, a cured product obtained by using the conductive composition is such that toughness can be improved, that is, both tensile strength and the degree of tensile elongation can be improved, and also flexibility can be improved.

When the conductive composition contains the plasticizer but does not contain the crosslinking agent, the degree of tensile elongation of a cured product obtained by using the conductive composition can be improved, and thus, as a whole, toughness of the cured product obtained by using the conductive composition can be improved. In addition, flexibility of the cured product obtained by using the conductive composition can be improved.

The conductive composition preferably contains both the crosslinking agent and the plasticizer. When the conductive composition contains both the crosslinking agent and the plasticizer, a cured product obtained using the conductive composition can have even more excellent toughness.

The conductive composition may contain, in addition to the above-described ingredients, any one or ones of various known additives such as a surfactant, a softening agent, a stabilizer, a leveling agent, an antioxidant, a hydrolysis inhibitor, a swelling agent, a thickener, a colorant, and a filler at an appropriate content rate as needed. Examples of the surfactant include silicone-based surfactants.

The conductive composition is prepared by mixing the above-described ingredients at the above-described ratio.

The conductive composition can contain a solvent at an appropriate content rate as necessary. Thereby, an aqueous solution of the conductive composition (conductive composition aqueous solution) is prepared.

As the solvent, an organic solvent or an aqueous solvent can be used. Examples of the organic solvent include ketones such as acetone and methyl ethyl ketone (MEK); esters such as ethyl acetate; ethers such as propylene glycol monomethyl ether; and amides such as N,N-dimethylformamide. Examples of the aqueous solvent include water; and alcohols such as methanol, ethanol, propanol, and isopropanol. Among them, the aqueous solvents are preferable.

The cured product obtained using the conductive composition has a pH of preferably 1 to 10, more preferably 1 to 8, and still more preferably 1 to 6. The pH of the cured product can be measured by any appropriate method, for example, a method in which litmus paper is brought into contact with the cured product, or a method in which a solution prepared by dissolving the conductive composition in a solvent is brought into contact with litmus paper.

An example of a method of producing the electrodes 30 using the conductive composition will be described.

The conductive polymer and the binder resin are mixed at the above-mentioned ratio to form a conductive composition containing the conductive polymer and the binder resin. The conductive composition may further contain at least one of the crosslinking agent or the plasticizer at the above-described rate. When the conductive composition is produced, the conductive polymer, the binder resin, and the crosslinking agent may be used as an aqueous solution obtained from being dissolved in a solvent.

The conductive composition may contain, if necessary, a solvent at an appropriate rate in addition to the solvent containing the conductive polymer, the binder resin, and the crosslinking agent, and thus, an aqueous solution of the conductive composition (a conductive composition aqueous solution) may be used. As the solvent, the same solvent as that described above can be used.

After the conductive composition is applied to a surface of a release base, the conductive composition is heated to cause a crosslinking reaction of the binder resin contained in the conductive composition to proceed, resulting in the binder resin being cured. As a result, a cured product of the conductive composition is obtained. If necessary, a surface of the obtained cured product is punched (pressed) using a press machine or the like to form one or more through-holes through the surface of the cured product and form the outer shape of the cured product into a predetermined shape. As a result, bioelectrodes, i.e., the electrodes 30, each of which is a shaped body having one or more through-holes through the surface thereof and having a predetermined outer shape is obtained. Instead of the press machine, a laser processing machine may be used for the shaping. In this regard, only the one or more through-holes may be formed through the surface of the obtained cured product, or only the outer shape may be made into a predetermined shape. In a case where the cured product can be used as the bioelectrode as it is, the cured product may be used as the bioelectrode without being subjected to a shaping process or the like.

Note that the conductive polymer, the binder resin, the crosslinking agent, and the plasticizer contained in the electrodes 30 have contents equivalent to those having been added at the time of the preparation of the conductive composition.

As the release base, a separator, a core material, or the like can be used. As the separator, a resin film such as a polyethylene terephthalate (PET) film, a polyethylene (PE) film, a polypropylene (PP) film, a polyamide (PA) film, a polyimide (PI) film, or a fluororesin film can be used. As the core material, a resin film such as a PET film or a PI film; a ceramic sheet; a metal film such as an aluminum foil; a resin substrate reinforced with a glass fiber or a plastic non-woven fiber; a silicone substrate or a glass substrate can be used.

Examples of a method of applying the conductive composition onto the release base include roll coating, screen coating, gravure coating, spin coating, reverse coating, bar coating, blade coating, air knife coating, dipping, dispensing, and the like, and a method in which a small amount of the conductive composition is dropped onto the base and then spread with a doctor blade. By any of these coating methods, the conductive composition is uniformly coated on the release base.

As a method of heating the conductive composition, a known dryer such as a drying oven, a vacuum oven, an air circulation type oven, a hot air dryer, a far infrared dryer, a microwave reduced pressure dryer, or a high frequency dryer can be used.

The heating condition should be a condition under which the crosslinking agent contained in the conductive composition can react.

The heating temperature with respect to the conductive composition is a temperature at which curing of the binder resin contained in the conductive composition can proceed. The heating temperature is preferably 100°C to 200°C. In the case where the conductive composition contains the crosslinking agent, when the heating temperature is in the range of 100°C to 200°C, reaction of the crosslinking agent easily proceeds and curing of the binder resin can be accelerated.

The heating time with respect to the conductive composition is preferably 0.5 minutes to 300 minutes, and more preferably 5 minutes to 120 minutes. When the heating time is in the range of 0.5 minutes to 300 minutes, the binder resin can be sufficiently cured.

Then, after the housing 10, the foam sheet 20, the electrodes 30, the supporting adhesive sheet 40, and the sensor unit 50 as elements of the living body sensor 1 depicted in FIG. 1 are prepared, the sensor unit 50 is placed on the supporting adhesive sheet 40. Thereafter, the housing 10, the foam sheet 20, the electrodes 30, and the supporting adhesive sheet 40 are stacked in this order from the housing 10 side toward the supporting adhesive sheet 40 side. Release paper 60 may be made to adhere to the attachment surface sides of the foam sheet 20 and the supporting adhesive sheet 40 with respect to the living body.

Thus, the living body sensor 1 depicted in FIG. 1 is obtained.

Thus, the living body sensor 1 includes the housing 10, the foam base 211, and the electrodes 30. Since the foam base 211 has a breaking elongation rate of 30% to 500%, the entire foam attachment layer 21 has appropriate flexibility and can be flexibly deformed to fit the contact surface with respect to skin 2. Therefore, when the living body sensor 1 is used by being attached to the skin 2, even if the skin 2 moves due to body motion or the like of the living body, the foam sheet 20 easily deforms following the movement of the skin 2 and the contact state with the skin 2 is easily maintained. Therefore, it is possible to suppress an increase in noise generated in an electrocardiogram. Since each of the electrodes 30 has a coefficient of static friction $\mu$ of 3.0 to 7.0, adhesiveness of the electrodes 30 to the skin 2 can be maintained. Therefore, the electrodes 30 are less likely to be displaced from the attachment surface with respect to the skin 2, and can be prevented from being peeled off from the skin 2. Therefore, an increase in noise generated in an electrocardiogram can be suppressed. Therefore, the living body sensor 1 can stably obtain an electrocardiographic waveform even when the subject is performing exercise and thus is moving.

In particular, in the living body sensor 1 having the above-described configuration, the electrodes 30 are provided on partial areas of the attachment side surface of the foam base 211 via the base adhesive layer 212, and the foam base 211 has the hole 211a substantially at the center thereof. Thus, it is important that the foam base 211 is easily deformed following movement of the skin 2, the living body sensor 1 is flexible, contact with the skin 2 is maintained, and the electrodes 30 can stably detect an electric signal. In the living body sensor 1, the breaking elongation rate of the foam base 211 is set to 30% to 500%, and thus the foam attachment layer 21 can be easily expanded and constricted. Therefore, even when the skin 2 moves, a state of the living body sensor 1 in which the attachment surface of the base adhesive layer 212 attached to the foam base 211 is stably attached to the skin 2 can be maintained. In addition, in the living body sensor 1, the coefficient of static friction μ of the electrodes 30 is set to 3.0 to 7.0, and it is possible to suppress shift of the attachment positions of the electrodes 30. Therefore, the living body sensor 1 can reduce the magnitude of a noise generated in an electrocardiogram even when a subject moves due to exercise or the like at a time of use, and thus can stably measure biological information from the skin 2 with high accuracy.

In addition, in the living body sensor 1, since the foam base 211 has the breaking elongation of 30% to 500%, it is possible to reduce a volume of a void generated inside the foam base 211, and thus it is possible to suppress intrusion of moisture from the outside. Therefore, durability of the living body sensor 1 can be improved.

Furthermore, in the living body sensor 1, since the electrodes 30 have the coefficient of static friction μ of 3.0 to 7.0, it is possible to reduce a burden to the skin with the electrodes 30 and to reduce influence to the skin such as skin chapping or skin rash. Therefore, the living body sensor 1 can be safely used even if it is attached to the subject for a long time.

The living body sensor 1 can include the housing adhesive layer 22 on the surface of the foam attachment layer 21 at the housing 10 side, i.e., the upper surface of the foam attachment layer 21 that includes the foam base 211. As a result, even when the subject moves and the skin 2 stretches, adhesion of the foam attachment layer 21 to the housing 10 is maintained, and production of a gap or the like between the foam attachment layer 21 and the housing 10 can be suppressed. Therefore, it is possible to prevent intrusion of moisture into the inside of the foam attachment layer 21. Therefore, in the living body sensor 1, deterioration of the foam attachment layer 21 can be suppressed, and thus durability can be maintained.

The living body sensor 1 includes the base adhesive layer 212, the base 41, and the sensor body 52; the housing 10 has the recess 11a formed in the indented shape at the skin 2 side; the foam base 211 has the hole 211a at the position corresponding to the recess 11a; and thus, the housing space S can be formed by the recess 11a and the hole 211a. Accordingly, although the living body sensor 1 includes the sensor body 52 therein, the base adhesive layer 212 can be prevented from being peeled off from the skin 2, and the state of being attached to the skin 2 can be maintained.

The living body sensor 1 includes the attachment adhesive layer 42, and thus, the attachment surface with respect to the living body can be formed by the base adhesive layer 212 and the attachment adhesive layer 42. Accordingly, in the living body sensor 1, although the electrodes 30 are attached to the attachment adhesive layer 42, a sufficient area of being attached to the skin 2 can be secured. Therefore, the attachment adhesive layer 42 can be suppressed from being peeled off from the skin 2, and a state in which the electrodes 30 are stably attached to the skin 2 can be maintained. Therefore, even if the living body sensor 1 is attached to the skin 2 of the subject for a long time, the living body sensor 1 can reliably detect biological information from the skin 2 during use.

In the living body sensor 1, the through holes 31 may be provided in the electrodes 30. As a result, it is possible to expose the base adhesive layer 212 to the attachment side through the through holes 31, and thus, it is possible to improve adhesion between the electrodes 30 and the skin 2. Therefore, the living body sensor 1 is such that, although the electrodes 30 are attached to the base adhesive layer 212, it is possible to prevent the base adhesive layer 212 from being peeled off from the skin 2, and it is possible to maintain a state in which the electrodes 30 are stably attached to the skin 2.

The living body sensor 1 can use the foam base 211 as a base. Since the foam base 211 has the porous structure, the living body sensor 1 easily follows movement of the skin 2, thus it is possible to suppress an increase in noise generated in an electrocardiographic waveform, and also, it is possible to reduce discomfort given to the subject. In addition, since water vapor due to sweat or the like generated from the skin 2 can be more reliably allowed to pass to the outside of the living body sensor 1 through the foam base 211, it is reliably possible to more easily maintain durability of the foam base 211 of the living body sensor 1.

Thus, the living body sensor 1 can be effectively used as an attachment type living body sensor which is attached to skin 2 or the like of a living body. As described above, the living body sensor 1 can hardly have positional deviation of the attachment surface with respect to the skin 2 during use, and also, has high durability, and it is possible to reduce a burden to the skin 2. Therefore, for example, the living body sensor 1 is attached to skin or the like of a living body, has a high advantageous effect of suppressing generation of noise in an electrocardiogram, and can be suitably used as a wearable device for health care requiring safety for skin.

[Examples]

Hereinafter, the embodiment will be described more specifically with reference to Examples and Comparative examples, but the embodiment is not limited to these Examples and Comparative examples.

<Production and Evaluation of Electrode A>

[Preparation of Conductive Composition]

0.38 parts by mass of PEDOT/PSS pellets ("Orgacon DRY", manufactured by Nippon AGFA Materials Japan. Ltd.) as the conductive polymer, 10.00 parts by mass of the aqueous solution containing modified polyvinyl alcohol (modified PVA) (modified polyvinyl alcohol concentration: 10%, "GOHSENX Z-410", manufactured by Nippon Synthetic Chemical Industry Co., Ltd.) as the binder resin, 2.00 parts by mass of glycerin (manufactured by Wako Pure Chemical Corporation) as the plasticizer, and 1.60 parts by mass of 2-propanol and 6.50 parts by mass of water as the solvent, were added to an ultrasonic bath. An aqueous solution containing these ingredients was mixed in the ultrasonic bath for 30 minutes to prepare a uniform conductive composition aqueous solution A.

Since the concentration of the modified PVA in the aqueous solution containing the modified PVA is about 10%, the content of the modified PVA in the conductive composition aqueous solution A is 1.00 parts by mass. The remainder is solvent in the conductive composition aqueous solution A.

The contents of the conductive polymer, the binder resin, and the plasticizer with respect to 100.0 parts by mass of the conductive composition were 11.2 parts by mass, 29.6 parts by mass, and 59.2 parts by mass, respectively.

[Production of Electrode Sheet]

The prepared conductive composition aqueous solution A was applied onto a polyethylene terephthalate (PET) film using an applicator. Thereafter, the PET film coated with the conductive composition aqueous solution A was conveyed to a drying oven (SPHH-201, manufactured by ESPEC CORP.), and the conductive composition aqueous solution A was heated and dried at 135°C for 3 minutes to prepare a cured product of the conductive composition. The cured product was punched (pressed) into a desired shape to form a sheet, thereby producing an electrode A that is an electrode sheet (bioelectrode).

The contents of the conductive polymer, the binder resin, and the plasticizer contained in the electrode A were the same as those in the conductive composition, and were 11.2 parts by mass, 29.6 parts by mass, and 59.2 parts by mass, respectively.

[Evaluation of Electrode A]

The coefficient of static friction $\mu$ of the obtained electrode A was measured.

(Measurement of Coefficient of Static Friction $\mu$)

The electrode A was cut to a size of 35 mm by 70 mm by 20 $\mu$m to prepare an electrode-sheet sample. Next, the coefficient of static friction $\mu$ of the electrode A with respect to a pseudo skin was measured as follows using a coefficient of static friction measuring device (TRIBOGEAR, type: 10, manufactured by SHINTO Scientific Co., Ltd.): The pseudo-skin used for the evaluation was a BIOSKIN plate (manufactured by Beaulax Co., Ltd., product number: P001-001, longitudinal size: 195 mm by lateral size: 120 mm by thickness: 5 mm) in which a surface of a urethane elastomer film is processed to reproduce hydrophilic/hydrophobic properties and surface wrinkles similar to those of a human skin. Then, the BIOSKIN plate was fixed to an elevating plate in a horizontal state, and the electrode sheet sample was attached to a planar indenter of longitudinal size: 75 mm by lateral size: 35 mm, the planar indenter was left at rest for 30 seconds under the condition of a load of 1.47 N, thereafter the planar indenter was inclined at an elevating speed of 10 degrees/6 seconds on average, a friction angle $\theta d$ when the planar indenter started to slide was read, and the coefficient of static friction $\mu$ was calculated based on the following formula (I):

$$\text{coefficient of static friction } \mu = \tan(\theta d \times \pi / 180) \quad \cdots \quad (I)$$

The content of each ingredient contained in the conductive composition aqueous solution A and the drying temperature are depicted in Table 1, and the content of each ingredient contained in the electrode A and the coefficient of static friction are depicted in Table 2.

[Preparation and Evaluation of Electrode B to Electrode f]

Preparation and evaluation of electrodes B to f were performed in the same manner as that of the above-described <Preparation and Evaluation of Electrode A> except that the conductive composition aqueous solutions B to f were used and the drying temperature was changed to those depicted in Table 1 to prepare the electrodes B to f.

The content of each ingredient contained in each of the conductive composition aqueous solutions B to f and the drying temperatures are depicted in Table 1, and the content of each ingredient contained in each of the electrodes B to f and the coefficients of static friction are depicted in Table 2.

[Table 1]

| | CONSTITUENTS (PARTS BY MASS) | | | | | | | DRYING TEMPERATURE [°C] |
|---|---|---|---|---|---|---|---|---|
| | CONDUCTIVE COMPOSITION | | | | | SOLVENT | | |
| | CONDUCTIVE POLYMER | BINDER RESIN | PLASTICIZER | TOTAL | REMAINDER | 2-PROPANOL | WATER | |
| CONDUCTIVE COMPOSITION AQUEOUS SOLUTION A | 0.38 (11.2) | 1.00 (29.6) | 2.00 (59.2) | 3.38 (100.0) | 96.62 (-) | 1.60 | 6.50 | 115 |
| CONDUCTIVE COMPOSITION AQUEOUS SOLUTION B | 0.38 (11.2) | 1.00 (29.6) | 2.00 (59.2) | 3.38 (100.0) | 96.62 (-) | 1.60 | 6.50 | 110 |
| CONDUCTIVE COMPOSITION AQUEOUS SOLUTION C | 0.38 (11.2) | 1.00 (29.6) | 2.00 (59.2) | 3.38 (100.0) | 96.62 (-) | 1.60 | 6.50 | 105 |
| CONDUCTIVE COMPOSITION AQUEOUS SOLUTION D | 0.38 (11.2) | 1.00 (29.6) | 2.00 (59.2) | 3.38 (100.0) | 96.62 (-) | 1.60 | 6.50 | 110 |
| CONDUCTIVE COMPOSITION AQUEOUS SOLUTION E | 0.38 (11.2) | 1.00 (29.6) | 2.00 (59.2) | 3.38 (100.0) | 96.62 (-) | 1.60 | 6.50 | 110 |
| CONDUCTIVE COMPOSITION AQUEOUS SOLUTION F | 0.38 (11.2) | 1.00 (29.6) | 2.00 (59.2) | 3.38 (100.0) | 96.62 (-) | 1.60 | 6.50 | 110 |
| CONDUCTIVE COMPOSITION AQUEOUS SOLUTION a | 0.38 (11.2) | 1.00 (29.6) | 2.00 (59.2) | 3.38 (100.0) | 96.62 (-) | 1.60 | 6.50 | 110 |
| CONDUCTIVE COMPOSITION AQUEOUS SOLUTION b | 0.38 (11.2) | 1.00 (29.6) | 2.00 (59.2) | 3.38 (100.0) | 96.62 (-) | 1.60 | 6.50 | 110 |
| CONDUCTIVE COMPOSITION AQUEOUS SOLUTION c | 0.38 (11.2) | 1.00 (29.6) | 2.00 (59.2) | 3.38 (100.0) | 96.62 (-) | 1.60 | 6.50 | 125 |
| CONDUCTIVE COMPOSITION AQUEOUS SOLUTION d | 0.38 (11.2) | 1.00 (29.6) | 2.00 (59.2) | 3.38 (100.0) | 96.62 (-) | 1.60 | 6.50 | 95 |
| CONDUCTIVE COMPOSITION AQUEOUS SOLUTION e | 0.38 (11.2) | 1.00 (29.6) | 2.00 (59.2) | 3.38 (100.0) | 96.62 (-) | 1.60 | 6.50 | 125 |
| CONDUCTIVE COMPOSITION AQUEOUS SOLUTION f | 0.38 (11.2) | 1.00 (29.6) | 2.00 (59.2) | 3.38 (100.0) | 96.62 (-) | 1.60 | 6.50 | 95 |

[Table 2]

| | COMPOSITION (PARTS BY MASS) | | | COEFFICIENT OF STATIC FRICTION μ |
|---|---|---|---|---|
| | CONDUCTIVE POLYMER | BINDER RESIN | PLASTICIZER | |
| ELECTRODE A | 11.2 | 29.6 | 59.2 | 3.5 |
| ELECTRODE B | 11.2 | 29.6 | 59.2 | 4.2 |
| ELECTRODE C | 11.2 | 29.6 | 59.2 | 5.7 |
| ELECTRODE D | 11.2 | 29.6 | 59.2 | 4.2 |
| ELECTRODE E | 11.2 | 29.6 | 59.2 | 4.2 |
| ELECTRODE F | 11.2 | 29.6 | 59.2 | 4.2 |
| ELECTRODE a | 11.2 | 29.6 | 59.2 | 4.2 |
| ELECTRODE b | 11.2 | 29.6 | 59.2 | 4.2 |
| ELECTRODE c | 11.2 | 29.6 | 59.2 | 2.5 |
| ELECTRODE d | 11.2 | 29.6 | 59.2 | 7.1 |
| ELECTRODE e | 11.2 | 29.6 | 59.2 | 2.5 |
| ELECTRODE f | 11.2 | 29.6 | 59.2 | 7.1 |

<Production and Evaluation of Foam A>

[Preparation of Foam A]

A polyolefin foam sheet ("Folec (registered trademark)", manufactured by INOAC CORPORATION, 0.5 mm in thickness) formed in a rectangular shape was foamed and expanded three times to obtain a foam A as a sheet-like foam base. The materials and the expansion ratios of the polyolefin foam sheets are depicted in Table 3.

[Measurement of Breaking Elongation Rate in Major Axis Direction of Foam A]

The breaking elongation rate in the major axis direction of the foam A was measured using a tensile tester (AGS-J, manufactured by Shimadzu Corporation). As a sample of the foam A, a rectangular sheet having a size of short side 10 mm by long side 70 mm by thickness 0.5 mm was used, and both end portions at the short sides of the sample of the foam A were gripped and fixed by tensile test jigs. The tensile test conditions were as follows: Three foams A were prepared, and the average value of the measured values was taken as the breaking elongation rate in the major axis direction of the foam A. The breaking elongation rate in the major axis direction of the foam A is depicted in Table 3.

(Tensile Test Conditions)

Width of foam A (maximum width in minor axis of foam A): 10 mm

Distance between jigs on which foam A is set: 50 mm Tensile strength: 300 mm/min

<Production and Evaluation of Foams B to f>

Production and evaluation of foams B to f were performed in the same manner as that of the above-described <Production and Evaluation of Foam A> except that the foams B to f were prepared with the changed expansion ratios of the polyolefin foam sheet having the values depicted in Table 3, and the breaking elongation rate in the major axis direction of each foam was measured. The thickness of each foam sample was appropriately set to a suitable size.
The material and the expansion ratio of each polyolefin foam sheet used in producing each of the foams B to f and the breaking elongation rate in the major axis direction of each of the foams B to f are depicted in Table 3.

[Table 3]

| TYPE | MATERIAL | EXPANSION RATIO [TIMES] | BREAKING ELONGATION RATE [%] |
|---|---|---|---|
| FOAM A | POLYOLEFIN FOAM SHEET | 3 | 36 |
| FOAM B | POLYOLEFIN FOAM SHEET | 4 | 43 |
| FOAM C | POLYOLEFIN FOAM SHEET | 6 | 62 |
| FOAM D | POLYOLEFIN FOAM SHEET | 15 | 158 |
| FOAM E | POLYOLEFIN FOAM SHEET | 25 | 265 |
| FOAM F | POLYOLEFIN FOAM SHEET | 45 | 487 |
| FOAM a | POLYOLEFIN FOAM SHEET | 2 | 24 |
| FOAM b | POLYOLEFIN FOAM SHEET | 55 | 545 |
| FOAM c | POLYOLEFIN FOAM SHEET | 15 | 158 |
| FOAM d | POLYOLEFIN FOAM SHEET | 15 | 158 |
| FOAM e | POLYOLEFIN FOAM SHEET | 2 | 24 |
| FOAM f | POLYOLEFIN FOAM SHEET | 55 | 545 |

<Example 1>

[Production of Living Body Sensor]

(Production of Housing)

A coat layer made of silicone rubber and having a Shore hardness A value of 40 was formed on a support made of PET as a base resin, and the thus obtained product was shaped into a predetermined shape to prepare the housing.

(Production of Foam sheet)

The base adhesive layer (a long-term attachment tape 1 (manufactured by Nitto Denko Corporation, 70 um thick)) was formed on the lower surface of the foam base 1 (polyolefin foam sheet ("Folec (registered trade mark)", manufactured by INOAC CORPORATION, 0.5 mm thick)) formed in a rectangular shape to form the foam attachment layer. The long-term attachment tape 1 was a double-sided adhesive tape having a wavy pattern (web pattern) formed on a surface thereof in which the width of each adhesive-formed portion having adhesive is about 500 um and the width of each no-adhesive portion having no adhesive is about 1500 um. Thereafter, the housing adhesive layer (a tape for silicone 1 ("ST503(HC)60", manufactured by Nitto Denko Corporation, 60 um thick)) was formed on the upper surface of the attachment layer to produce the foam sheet.

(Production of Supporting Adhesive Sheet)

An adhesive 1 ("PERME-ROLL" manufactured by Nitto Denko Corporation, moisture permeability: 21 (g/m$^2$·- day)), as the attachment adhesive layer and the sensor adhesive layer, was attached to each of both surfaces of a rectangular base 1 (PET ("PET-50-SCA1 (white)" manufactured by Mitsui & Co. Plastics Ltd.), 38 um thick) to prepare the supporting adhesive sheet that is a tape for skin.

(Production of Living Body Sensor)

The sensor unit including the battery and the control unit was installed at the center of the upper surface of the supporting adhesive sheet. Thereafter, the pair of electrodes were attached to the attachment surface side of the base adhesive layer in a state of being sandwiched between the base adhesive layer of the foam sheet and the supporting adhesive sheet; and the electrodes and wires of the sensor unit were connected. Thereafter, the housing was placed on the foam sheet so that the sensor unit was disposed in the housing space formed by the foam sheet and the housing. Thus, the living body sensor was produced.

[Characteristic Evaluation of Living Body Sensor]

As characteristics of the obtained living body sensor, noise, influence on skin, and durability of the living body sensor were evaluated.

(Evaluation of Noise)

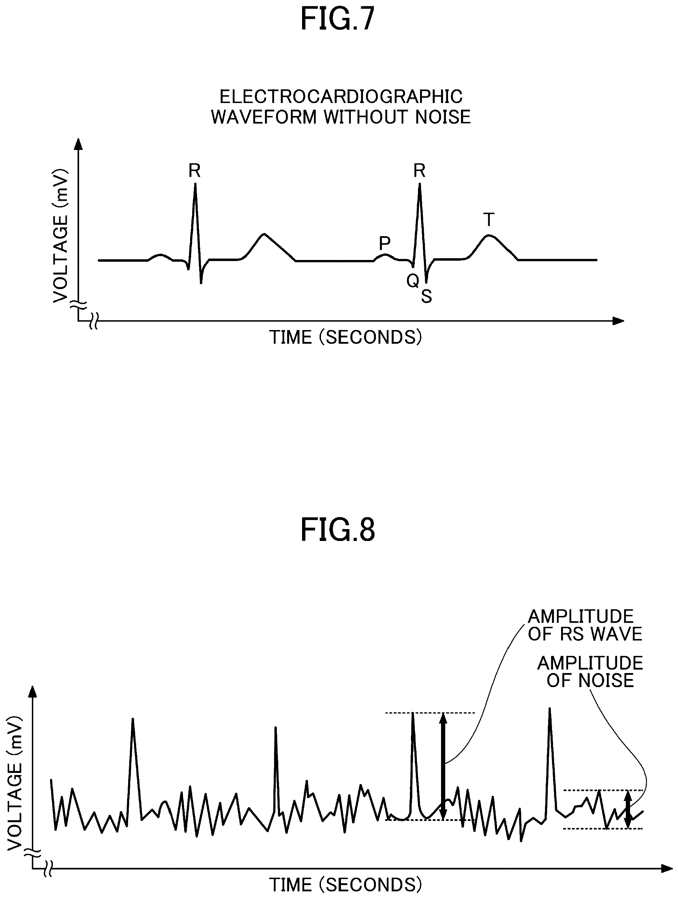

The obtained living body sensor was attached to skin of a subject for 24 hours to measure an electrocardiogram, and an electrocardiographic waveform was obtained. When there is no noise, the electrocardiographic waveform includes a P wave, a QRS wave, and a T wave as depicted in FIG. 7. As depicted in FIG. 8, from the obtained electrocardiographic waveform, the magnitude of the amplitude of a RS wave including a R wave and a S wave included in the QRS wave was determined as a signal (S); and the magnitude of the amplitude of noise, which is the amplitude of the waveform present between adjacent R waves, was determined as noise (N). Then, a SN ratio which is a ratio of the signal (S) to the noise (N) was obtained. As the SN ratio, a value obtained by extracting any three waveforms and averaging respective values obtained from these extracted waveforms was used.
The obtained SN ratio was evaluated based on the following evaluation criteria, and thus, the noise of the living body sensor was evaluated: When the SN ratio was greater than or equal to 8, it was evaluated that there was almost no noise during walking (indicated as A in Table 4); when the SN ratio was greater than or equal to 5 and less than 8, it was evaluated that there was slight noise during walking (indicated as B in Table 4); when the SN ratio was greater than or equal to 1 and less than 5, it was evaluated that there was large noise during walking but the R wave was able to be detected (indicated as C in Table 4); and when the SN ratio was less than or equal to 1, it was evaluated that there was large noise during walking and the R wave was not able to be detected (indicated as D in Table 4) .

Evaluation Criteria

A: The SN ratio is 8 or more.

B: The SN ratio is 5 or more and less than 8.

C: The SN ratio is more than 1 and less than 5.

D: The SN ratio is 1 or less.

(Evaluation of Influence on Skin)

After attaching the living body sensor to the skin of the test subject for 24 hours of the above (measurement of noise), the living body sensor was peeled off from the skin, the state of the skin at the place where the living body sensor was attached was visually observed, and the influence on the skin was evaluated based on the following evaluation criteria: When no redness was observed in the skin's attachment portion and thus there was no problem, the evaluation result was determined as very good (indicated as A in Table 4). When slight redness was observed in the skin's attachment portion but there was no problem, the evaluation result was determined as good (indicated as B in Table 4). When redness was strongly observed in the skin's attachment portion or when skin chapping was observed in the skin's attachment portion to such an extent that the living body sensor could not be attached again was observed in the skin's attachment portion, the evaluation result was determined as bad (indicated as C or D in Table 4).

Evaluation criteria

A: No redness was observed in the skin's attachment portion, and there was no problem.
B: Slight redness was observed in the skin's attachment portion, but there was no problem.
C: Strong redness was observed in the skin's adhesion portion.
D: Skin chapping to such an extent that the living body sensor cannot be attached again is observed in the skin's attachment part.

(Evaluation of Durability)

In the above (measurement of noise), the living body sensor was attached to the skin of the test subject for 24 hours. In addition, while the living body sensor was attached to the skin of the test subject, the living body sensor was brought into contact with water based on "the waterproof standard: JIS C 0920-1993 (IPX4)". After the living body sensor was attached to the skin of the test subject for 24 hours, the living body sensor was peeled off from the skin, the state of the living body sensor was observed, and durability was evaluated based on the following evaluation criteria: The living body sensor was determined to be excellent (indicated as A in Table 4) when neither water absorption nor breakage was observed in the living body sensor and there was no problem. The living body sensor was determined to be very good (indicated as B in Table 4) when there was no problem in the range of use; some water absorption or breakage was observed in the living body sensor but the influence on measurement was limited. The living body sensor was determined to be good (indicated as C in Table 4) when peeling or the like occurred due to water absorption or a breakage, but the measurement was able to be performed for 24 hours. The living body sensor was determined to be bad (indicated as D in Table 4) when peeling or the like occurred due to water absorption or a breakage during the measurement, and therefore, the measurement for 24 hours was not able to be performed.

Evaluation criteria

A: Water absorption or a breakage is not observed, and there is no problem.
B: There is no problem in the range of use; some absorption of water or breakage is observed, but influence on the measurement is limited.
C: Peeling or the like occurs due to absorption of water or a breakage, but the measurement can be performed for 24 hours.
D: Peeling or the like occurs due to absorption of water or a breakage, and the measurement for 24 hours cannot be performed.

<Examples 2 to 6 and Comparative Examples 1 to 6>

The test was carried out in the same manner as in Example 1 except that the types of the electrode and the foam used were changed.
Table 3 depicts the types of the electrodes and the foams used and the results of characteristics of the living body sensor in the Examples and Comparative examples are depicted in Table 3.

[Table 4]

| | LIVING BODY SENSOR | | CHARACTERISTICS | | |
|---|---|---|---|---|---|
| | ELECTRODE | FOAM | NOISE | INFLUENCE ON SKIN | DURABILITY |
| EXAMPLE 1 | ELECTRODE A | FOAM A | B | A | A |
| EXAMPLE 2 | ELECTRODE B | FOAM B | B | A | A |
| EXAMPLE 3 | ELECTRODE C | FOAM C | A | B | A |
| EXAMPLE 4 | ELECTRODE D | FOAM D | A | A | A |
| EXAMPLE 5 | ELECTRODE E | FOAM E | A | A | B |
| EXAMPLE 6 | ELECTRODE F | FOAM F | B | A | B |

(continued)

| | LIVING BODY SENSOR | | CHARACTERISTICS | | |
|---|---|---|---|---|---|
| | ELECTRODE | FOAM | NOISE | INFLUENCE ON SKIN | DURABILITY |
| COMPARATIVE EXAMPLE 1 | ELECTRODE a | FOAM a | C | A | A |
| COMPARATIVE EXAMPLE 2 | ELECTRODE b | FOAM b | C | A | C |
| COMPARATIVE EXAMPLE 3 | ELECTRODE c | FOAM c | C | A | A |
| COMPARATIVE EXAMPLE 4 | ELECTRODE d | FOAM d | B | C | A |
| COMPARATIVE EXAMPLE 5 | ELECTRODE e | FOAM e | D | A | A |
| COMPARATIVE EXAMPLE 6 | ELECTRODE f | FOAM f | C | C | C |

From Table 4, it was confirmed that, in each of the Examples 1 to 6, all of the noise, the influence on skin, and durability of the living body sensor satisfied the conditions for use. On the other hand, with regard to each of the Comparative examples 1 to 6, it was confirmed that at least one or more of the noise, the influence on skin, and durability of the living body sensor did not satisfy the conditions for use, and there was a problem in practical use.

Therefore, unlike the living body sensors of the Comparative examples 1 to 6, with regard to each of the living body sensors of the Examples 1 to 6, it was possible to suppress noise generated in an electrocardiogram and stably obtain an electrocardiographic waveform even when the subject was performing exercise, as a result of the foam base having the breaking elongation rate of 30% to 500% and the electrodes having the coefficient of static friction $\mu$ of 3.0 to 7.0 being included, and a burden to skin was able to be reduced while durability was able to be improved. Therefore, it can be said that even if the bioelectrodes according to the present embodiment were attached to skin of the subject for a long time (for example, 24 hours), the bioelectrodes were able to be effectively used for stably measuring an electrocardiogram without causing a burden to the subject continuously for the long time.

While the embodiments have been thus described, these embodiments have been presented by way of example only, and are not intended to limit the scope of the present invention. The above-described embodiments can be implemented in various other forms, and various combinations, omissions, replacements, changes, and the like can be made without departing from the scope of the invention. These embodiments and modifications thereof are included in the scope of the invention, and are included in the invention described in the claims and the scope equivalent thereto.

The present application claims priority based on Japanese Patent Application No. 2020-192638 filed with the Japan Patent Office on November 19, 2020 and Japanese Patent Application No. 2021-174265 filed with the Japan Patent Office on October 26, 2021, and the entire contents of Japanese Patent Application No. 2020-192638 and Japanese Patent Application No. 2021-174265 are incorporated herein by reference.

[Description of Symbols]

1 living body sensor
2 skin
10 housing
20 foam sheet
21 foam attachment layer
211 foam base
211a hole
212 base adhesive layer
22 housing adhesive layer
30 electrode
31 through-hole
40 supporting adhesive sheet
41 base
42 attachment adhesive layer
43 sensor adhesive layer
50 sensor unit
51 flexible substrate (resin substrate)

52      sensor body
54      battery

**Claims**

1.  A living body sensor configured to be attached to a living body to obtain a biological signal, the living body sensor comprising:

    a housing;
    a base provided on a living body side of the housing; and
    an electrode provided at a surface of the base on a living body side,
    wherein
    the base has a breaking elongation rate of 30% to 500%, and
    the electrode has a coefficient of static friction of 3.0 to 7.0.

2.  The living body sensor as claimed in claim 1,
    wherein
    a housing adhesive layer is provided at a surface of a foam attachment layer on a housing side, the foam attachment layer including the base.

3.  The living body sensor as claimed in claim 1 or 2, comprising:

    a base adhesive layer provided at a surface of the base on a living body side, the electrode being attached to the base adhesive layer;
    a sensor body connected to the electrode and configured to obtain biological information; and
    a base for mounting the sensor body,
    wherein
    the housing has a recess formed in an indented shape on a living body side, and
    the base has a hole at a position corresponding to the recess, and
    a housing space in which the sensor body is housed is formed by the recess and the hole.

4.  The living body sensor as claimed in claim 3, comprising

    an attachment adhesive layer provided on a living body side of the base,
    wherein
    the base adhesive layer and the attachment adhesive layer form an attachment surface with respect to the living body.

5.  The living body sensor as claimed in claim 3 or 4,
    wherein
    the electrode has a through hole through which the base adhesive layer is exposed in a state where the electrode is attached to the base adhesive layer.

6.  The living body sensor as claimed in any one of claims 1 to 5, wherein the base is a foam base having a porous structure.

7.  The living body sensor according to any one of claims 1 to 6,
    wherein
    the breaking elongation rate of the base is measured by gripping both ends at short sides of a sheet of the base having a size of short side 10 mm by long side 70 mm by thickness 0.5 mm with tensile test jigs and pulling the sheet at a tensile strength of 300 mm/min.

8.  The living body sensor as claimed in any one of claims 1 to 7,
    wherein
    the coefficient of static friction of the electrode is measured by setting a planar indenter to which the electrode is attached onto a pseudo skin with a load of 1.47 N, thereafter inclining the planar indenter, and substituting a friction angle $\theta d$ measured at a time when the planar indenter starts sliding to a formula (I):

coefficient of static friction μ

$$= \tan(\theta d \times \pi / 180) \qquad \cdots \quad (\mathrm{I})$$

# FIG.1

# FIG.2

# FIG.3

EP 4 248 868 A1

# FIG.4

EP 4 248 868 A1

# FIG.5

# FIG.6

# FIG.7

ELECTROCARDIOGRAPHIC
WAVEFORM WITHOUT NOISE

# FIG.8

## INTERNATIONAL SEARCH REPORT

| International application No. |
| --- |
| **PCT/JP2021/041854** |

### A.    CLASSIFICATION OF SUBJECT MATTER

*A61B 5/332*(2021.01)i
FI:    A61B5/332

According to International Patent Classification (IPC) or to both national classification and IPC

### B.    FIELDS SEARCHED

Minimum documentation searched (classification system followed by classification symbols)

A61B5/25-5/398

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C.    DOCUMENTS CONSIDERED TO BE RELEVANT

| Category* | Citation of document, with indication, where appropriate, of the relevant passages | Relevant to claim No. |
| --- | --- | --- |
| P, Y | JP 6947955 B1 (NITTO DENKO CORP.) 13 October 2021 (2021-10-13) paragraphs [0011]-[0068], fig. 1-5 | 1-5, 7-8 |
| P, A | | 6 |
| Y | JP 2020-163128 A (NITTO DENKO CORP.) 08 October 2020 (2020-10-08) paragraphs [0120]-[0121] | 1-8 |
| Y | JP 2019-150465 A (NITTO DENKO CORP.) 12 September 2019 (2019-09-12) paragraphs [0040]-[0041] | 1-8 |
| Y | WO 2019/044649 A1 (TOYOBO CO., LTD.) 07 March 2019 (2019-03-07) claim 3 | 1-8 |
| Y | JP 2013-531512 A (IRHYTHM TECHNOLOGIES, INC.) 08 August 2013 (2013-08-08) claims 59-66, paragraph [0026] | 1, 6-8 |
| A | WO 2019/188311 A1 (NIPRO CORP.) 03 October 2019 (2019-10-03) entire text, all drawings | 1-8 |

☐ Further documents are listed in the continuation of Box C.        ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **24 January 2022** | **01 February 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2021/041854**

| Patent document cited in search report | | | Publication date (day/month/year) | Patent family member(s) | | Publication date (day/month/year) |
|---|---|---|---|---|---|---|
| JP | 6947955 | B1 | 13 October 2021 | (Family: none) | | |
| JP | 2020-163128 | A | 08 October 2020 | WO 2020/196099 A1 | | |
| JP | 2019-150465 | A | 12 September 2019 | (Family: none) | | |
| WO | 2019/044649 | A1 | 07 March 2019 | US 2020/0221965 A1 claim 3 EP 3677176 A1 CN 110996783 A KR 10-2020-0044842 A | | |
| JP | 2013-531512 | A | 08 August 2013 | US 2011/0279963 A1 claims 59-66, paragraph [0026] WO 2011/143490 A2 EP 2568878 B1 AU 2011252998 A1 CA 2797980 A1 KR 10-2013-0045271 A DK 2568878 T3 ES 2692658 T3 | | |
| WO | 2019/188311 | A1 | 03 October 2019 | US 2021/0007623 A1 entire text, all drawings EP 3777670 A1 CN 111902081 A | | |

Form PCT/ISA/210 (patent family annex) (January 2015)

**EP 4 248 868 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019044649 A
- JP 2016166436 A
- JP 2020192638 A
- JP 2021174265 A